# EUROPÄISCHE PATENTANMELDUNG

(11) **EP 4 596 596 A1**
(43) Veröffentlichungstag der Anmeldung: **06.08.2025**
(21) Anmeldenummer: 25154365.8
(22) Anmeldetag: 28.01.2025
(51) Int. Cl.: C08G 18/09, C08G 18/16, C08G 18/18, C08G 18/22, C08G 18/42, C08G 18/48, C08G 18/76, C08J 9/14

(54) **HERSTELLUNG VON POLYURETHAN- ODER POLYISOCYANURAT-SCHAUMSTOFF**

(30) Priorität: 02.02.2024 EP 24155463
(71) Anmelder: Evonik Operations GmbH, 45128 Essen (DE)
(72) Erfinder: Glos, Martin, 46325 Borken (DE)
(74) Vertreter: Evonik Patent Association

(57) **Zusammenfassung**

Die vorliegende Erfindung betrifft eine Zusammensetzung zur Herstellung von Polyurethan- oder Polyisocyanurat-Schaumstoff, umfassend eine Polyisocyanat-Komponente, eine Polyolkomponente, mindestens ein tertiäres Amin der Formel (X), sowie mindestens ein Zink(II)-Carboxylat, mit der Maßgabe, dass das enthaltene Zink(II)-Carboxylat in stöchiometrischer Form eingesetzt wird, also mit Zn(II) und Carboxylat im molaren Verhältnis von 1 zu 2, wobei die Zusammensetzung zusätzlich mindestens eine Stickstoff-haltige Verbindung V enthält, welche mindestens zwei N-Atome aufweist und bei der es sich um ein modifiziertes Phenol handelt.

## Beschreibung

Die vorliegende Erfindung liegt auf dem Gebiet der Polyurethane (PU) und Polyisocyanurate (PIR), insbesondere der PU- oder PIR-Schaumstoffe. Insbesondere betrifft sie eine Zusammensetzung zur Herstellung von Polyurethan- oder Polyisocyanurat-Schaumstoff, eine Zink(II)-Carboxylat-haltige Zubereitung, geeignet zur Katalyse bei der Herstellung von PU- oder PIR-Schaumstoff, ein Verfahren zur Herstellung von Polyurethan- oder Polyisocyanurat-Schaumstoff, sowie Polyurethan- oder Polyisocyanurat-Schaumstoff und weiterhin die Verwendung der Schaumstoffe, die damit hergestellt wurden.

Unter Polyurethan (PU) wird im Rahmen der vorliegenden Erfindung insbesondere ein Produkt, erhältlich durch Reaktion von Polyisocyanaten und Polyolen bzw. Verbindungen mit Isocyanat-reaktiven Gruppen, verstanden. Es können hierbei neben dem Polyurethan auch weitere funktionelle Gruppen gebildet werden, wie z.B. Uretdione, Carbodiimide, Isocyanurate, Allophanate, Biurete, Harnstoffe und/oder Uretimine. Daher werden unter PU im Sinne der vorliegenden Erfindung sowohl Polyurethan als auch Polyisocyanurat, Polyharnstoffe und Uretdion-, Carbodiimid-, Allophanat-, Biuret- und Uretimin-Gruppen enthaltende Polyisocyanat-Reaktionsprodukte verstanden. Unter Polyurethanschaumstoff (PU-Schaumstoff) wird im Rahmen der vorliegenden Erfindung insbesondere Schaumstoff verstanden, der als Reaktionsprodukt basierend auf Polyisocyanaten und Polyolen bzw. Verbindungen mit Isocyanat-reaktiven Gruppen erhalten wird. Es können hierbei neben dem Namen gebenden Polyurethan auch weitere funktionelle Gruppen gebildet werden, wie z.B. Allophanate, Biurete, Harnstoffe, Carbodiimide, Uretdione, Isocyanurate oder Uretimine. Unter Polyisocyanuratschaumstoff (PIR-Schaumstoff) wird im Rahmen der vorliegenden Erfindung insbesondere ein Schaumstoff verstanden, der als Reaktionsprodukt basierend auf Polyisocyanaten und Polyolen bzw. Verbindungen mit Isocyanat-reaktiven Gruppen erhalten wird und bei denen der Isocyanat-Index, also das Verhältnis zwischen Isocyanat- und Isocyanat-reaktiven Gruppen vorzugsweise größer 180 ist. Es werden hierbei neben dem Namen gebenden Polyisocyanuraten in jedem Fall auch Polyurethan-Gruppen, sowie gegebenenfalls weitere funktionelle Gruppen, wie z.B. Allophanate, Biurete, Harnstoffe, Carbodiimide, Uretdione, oder Uretimine, gebildet.

Im Rahmen der vorliegenden Erfindung steht vorzugsweise die Bildung von Polyisocyanuraten (PIR) im Vordergrund. Diese Reaktion wird als Trimerisierung bezeichnet, da formal drei Isocyanat-Gruppen zu einem Isocyanurat-Ring reagieren. Die Herstellung von z.B. PIR-Hartschaumstoff ist in der Literatur beschrieben und erfolgt vorzugsweise durch Umsetzung von Polyisocyanaten mit Verbindungen mit gegenüber Isocyanatgruppen reaktiven Wasserstoffatomen, zumeist Polyetherole, Polyesterole oder beide, wobei der Isocyanatindex vorzugsweise 180 und größer ist. Dadurch bilden sich zusätzlich zu den Urethanstrukturen, die durch die Umsetzung von Isocyanaten mit Verbindungen mit reaktiven Wasserstoffatomen entstehen, durch Reaktion der Isocyanatgruppen untereinander Isocyanuratstrukturen oder weitere Strukturen, die durch die Reaktion von Isocyanatgruppen mit anderen Gruppen, wie zum Beispiel Polyurethangruppen, entstehen.

Bei der Herstellung von Polyurethan- oder Polyisocyanurat-Schaumstoffen, vorzugsweise Polyurethan- oder Polyisocyanurat-Hartschaumstoffen können verschiedene Katalysatoren eingesetzt werden, um das Reaktionsprofil der Verschäumung und die Gebrauchseigenschaften des Schaumes positiv zu beeinflussen. Dabei ist die Bildung von Polyisocyanuraten vorteilhaft, da diese zu guten mechanischen Eigenschaften (hohe Stauchhärte) und verbesserten flammhemmenden Eigenschaften führen.

Verschiedene Veröffentlichungen bezüglich der Verwendung von Katalysatoren zur Verbesserung der Stauchhärte durch Unterstützung der Trimerisierungsreaktion bei der Herstellung von PU- oder PIR-Hartschäumen sind bekannt.

In EP 1878493 A1 wird die Verwendung von Carbokationenverbindungen als Trimerisierungskatalysatoren beschrieben, wobei die Anionen auf Di-Carbonylverbindungen basieren. Die Verwendung von Zink-Carboxylaten wird nicht beschrieben.

Die US 4,452,829 beschreibt die Herstellung von Sprühschaum unter Einsatz von Triolen mit Molmassen von über 1000 g/mol. Hierbei werden Zn-Salze in Kombination mit K-Salzen verwendet um das Cremen, also den Start der PU-Reaktion mit Wasser zu beschleunigen bzw. dort wird zu einem K-haltigen Katalysator noch ein Zn-haltiger Katalysator (Zink-Octoat) zugegeben, um die Cremezeit zu verkürzen, also die Reaktion zu beschleunigen.

US 4,200,699 beschreibt Gelkatalysator-Zusammensetzungen für die Herstellung von PU-Hartschäumen enthaltend Zn-, K- und Sn-Carboxylate, wobei vorzugsweise ein weiterer Gelkatalysator aus der Gruppe der tertiären Amine, der anorganischen Zinnverbindungen oder der OrganozinnVerbindungen eingesetzt wird.

Die EP 1745847 A1 beschreibt Trimerisierungskatalysatoren auf Basis von Kaliumoctoat und Lösungsmitteln, die gegen die Reaktion mit Isocyanaten inert sind.

In WO 2016/201675 A1 werden Trimerisierungskatalysatoren beschrieben, bestehend aus Zusammensetzungen basierend auf sterisch gehinderten Carboxylaten und tert. Aminen, die eine Isocyanat-reaktive Gruppe tragen.

WO 2010/054317 A2 beschreibt Imidazolium- oder Imdazolinium-Salze als Trimerisierungskatalysatoren.

Die WO 2013/074907 A1 beschreibt die Verwendung von Tetraalkylguanidin-Salzen aromatischer Carbonsäuren als Katalysatoren für Polyurethan-Schäume.

WO 2015/179041 A1 beschreibt die Verwendung von Zink-basierenden Katalysatoren zur Vernetzung von Silikon-Harzen. Hierbei werden unterschiedliche Liganden verwendet, unter anderem Phenol-basierende Liganden. Es wird aber keine Katalyse einer Polyurethan- bzw. IsocyanatReaktion beschrieben.

WO 2022/218657 A1 beschreibt die Herstellung von Polyurethan- oder Polyisocyanurat-Hartschaumstoff unter Einsatz von Zinksalzen und/oder einer Zink-haltigen Zubereitung.

US 2009/099274 A1 offenbart PU- oder PIR-Hartschaumstoff, hergestellt aus einer Zusammensetzung umfassend eine Isocyanat-Komponente, eine Polyol-Komponente, ein Treibmittel, Aminbasierenden Katalysator und Zink-Katalysator.

WO 2019/122923 A1 offenbart PU- oder PIR-Hartschaumstoff, hergestellt aus Polyisocyanat, Polyethercarbonat und Katalysator basierend auf Zink.

EP 0 010 407 A1 offenbart ein Verfahren zur Herstellung von PU-Schaum in Gegenwart von Gelkatalysatoren, welche Zink-Caboxylate enthalten.

Aufgabe der vorliegenden Erfindung war es, eine weitere Möglichkeit der Bereitstellung von Polyurethan- oder Polyisocyanurat-Schaumstoffen, vorzugsweise Polyurethan- oder Polyisocyanurat-Hartschaumstoffen, hervorzubringen, die besonders vorteilhafte Gebrauchseigenschaften, wie insbesondere gute Stauchhärte und/oder Eindrückhärte schon nach kurzer Reaktionszeit aufweisen. Dabei sollte allerdings der Einfluss auf das Steigprofil vorzugsweise möglichst gering gehalten werden.

Die Lösung dieser Aufgabe wird ermöglicht vom Gegenstand der Erfindung.

Der Gegenstand der Erfindung ist eine Zusammensetzung zur Herstellung von Polyurethan- oder Polyisocyanurat-Schaumstoff, vorzugsweise Polyurethan- oder Polyisocyanurat-Hartschaumstoff, umfassend
a) eine Polyisocyanat-Komponente,
b) eine Polyolkomponente,
c) mindestens ein Zink(II)-Carboxylat, mit der Maßgabe, dass das enthaltene Zink(II)-Carboxylat in stöchiometrischer Form eingesetzt wird, also mit Zn(II) und Carboxylat im molaren Verhältnis von 1 zu 2,
d) mindestens ein tertiäres Amin der Formel (X) 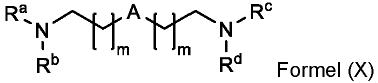 mit
   - m: jeweils unabhängig voneinander 1 oder 2,
   - A: ist O, S oder N-R^{e},
   - R^{a}, R^{b}, R^{c}, R^{d} und R^{e},: jeweils unabhängig voneinander gleiche oder verschiedene lineare, verzweigte oder cyclische Alkylreste mit 1 bis 20 Kohlenstoffen,
e) optional mindestens einen Schaumstabilisator,
f) optional mindestens ein Treibmittel,
   wobei die Zusammensetzung zusätzlich
g) mindestens eine Stickstoff-haltige Verbindung V enthält, welche mindestens zwei N-Atome aufweist und bei der es sich um ein modifiziertes Phenol handelt.

Es konnte gefunden werden, dass der Einsatz erfindungsgemäßer Zusammensetzungen bei der PU- oder PIR-Schaumstoffherstellung, vorzugsweise PU- oder PIR-Hartschaumstoffherstellung, zu entsprechenden Schaumstoffen, vorzugsweise Hartschaumstoffen, mit verbesserten Gebrauchseigenschaften führt. Insbesondere wird die Trimierisierung verbessert, wodurch die Schäume schnell aushärten, das heißt zu einem frühen Zeitpunkt schon eine hohe Stauchhärte und hohe Eindrückhärte aufweisen.

Ein besonderer Vorteil der Erfindung liegt weiterhin darin, dass der Einsatz der erfindungsgemäßen Zusammensetzungen es dennoch ermöglicht, dass der Einfluss auf das Steigprofil möglichst gering gehalten werden kann. Dies ist sehr vorteilhaft, da man sonst Probleme mit der Fließfähigkeit der Reaktionsmischung bekommen kann, was zu erheblichen Verarbeitungsproblemen führt.

Mit den erfindungsgemäßen Zusammensetzungen können die Steigprofile ggf. auch verlangsamt werden, was sehr vielseitige Möglichkeiten zur Anpassung der Reaktivität eines Schaum-Systems ermöglicht.

Mit der erfindungsgemäßen Lösung können somit PU- oder PIR-Schaumstoff-, vorzugsweise PU- oder PIR-Hartschaumstoff-basierte Produkte wie z.B. Isolationspanele oder Kühlmöbel mit ganz besonders hoher Qualität hergestellt und die Prozesse zur Herstellung der PU- oder PIR-Schaumstoffe, vorzugsweise PU- oder PIR-Hartschaumstoffe können effizienter gestaltet werden.

Ein zusätzlicher Vorteil der Erfindung ist die gute ökotoxikologische Einstufung der verwendbaren Chemikalien, insbesondere der Zink(II)-Carboxylate. Denn oftmals werden im Stand der Technik Metallverbindungen mit problematischen toxikologischen Eigenschaften eingesetzt (wie z.B. Sn, Pb, etc.).

Die Erfindung hat den weiteren Vorteil, dass mit ihrer Hilfe PU- bzw. PIR-Schaumstoffe, vorzugsweise PU- bzw. PIR-Hartschaumstoffe, hergestellt werden können, die wenig Schaumdefekte aufweisen.

Die Erfindung hat den weiteren Vorteil, dass hohe Wirk-Konzentrationen an Zink-Salzen in der Reaktionsmischung ermöglicht werden können. Es kann eine bessere Lösung des Zink-Salzes erfolgen, so dass beispielsweise die gesamt einzusetzende Menge einer Zink-haltiger Zubereitung, die einer Reaktionsmischung zugegeben wird, sogar reduziert werden kann.

Die erfindungsgemäße Zusammensetzung umfasst mindestens ein Zink(II)-Carboxylat, vorzugsweise ausgewählt aus der Gruppe bestehend aus Zink(II)-acetat, Zink(II)-propionat, Zink(II)-pivalat, Zink(II)-2-ethylhexanoat (Zink(II)-octoat), Zink(II)-isononanoat (Zink(II)-3,5,5-trimethyl-hexanoat), Zink(II)-neodecanoat, Zink(II)-ricinoleat, Zink(II)-palmitat, Zink(II)-stearat, Zink(II)-oleat, Zink(II)-laurat, Zink(II)-napthenat, Zink(II)-benzoat, Zink(II)-laktat, Zink(II)-glycinat, Zink(II)-hippurat, Zink(II)-citrat und Zink(II)-Seifen, wobei der Einsatz von Zink(II)-acetat und/oder Zink(II)-ricinoleat ganz besonders bevorzugt ist. Bevorzugt einsetzbare Zink(II)-Seifen sind Zinkoleat, Zinkpalmitat, und/oder Zinkstearat.

Das in der erfindungsgemäßen Zusammensetzung enthaltene Zink(II)-Carboxylat wird in stöchiometrischer Form eingesetzt, also mit Zn(II) und Carboxylat im molaren Verhältnis von 1 zu 2. Das bedeutet, dass kein Überschuss an (Carboxylat bzw). Carbonsäure eingesetzt wird oder vorliegt. Bei technischen Herstellverfahren von Zinksalzen werden die zugrunde liegenden Säuren mitunter im Überschuss eingesetzt, so dass das jeweilige Endprodukt noch einen Überschuss der Säure enthält. Ein entsprechender Überschuss der Säure ist für die vorliegende Erfindung nicht zweckdienlich.

Das in der erfindungsgemäßen Zusammensetzung enthaltene mindestens eine Zink(II)-Carboxylat kann in die betreffende Zusammensetzung auf jede bekannte Weise eingebracht werden, es ist allerdings bevorzugt, dass Zink(II)-Carboxylat in gelöster Form einzubringen, vorzugsweise unter Einsatz mindestens einer Stickstoff-haltigen Verbindung V und optional mindestens eines Trägermediums (d.h. Lösungsmittels) einzubringen. Grundsätzlich können als Trägermedien alle als Lösungsmittel geeigneten Substanzen optional verwendet werden. Bevorzugt können beispielweise Glykole, Alkoxylate und/oder Öle synthetischer und/oder natürlicher Herkunft optional zum Einsatz gelangen. Bevorzugt können protische oder aprotische Lösungsmittel optional verwendet werden.

Es ist ganz besonders bevorzugt, wenn das in der erfindungsgemäßen Zusammensetzung enthaltene mindestens eine Zink(II)-Carboxylat in Form einer Zink(II)-Carboxylat-haltigen Zubereitung in die Zusammensetzung eingebracht wird, wobei diese Zubereitung vorzugsweise mindestens eine erfindungsgemäße Stickstoff-haltige Verbindung V und optional mindestens ein Trägermedium umfasst. Eine entsprechende, besonders bevorzugt einsetzbare Zink(II)-Carboxylat-haltige Zubereitung ist weiter unten genauer beschrieben.

Die erfindungsgemäße Zusammensetzung enthält mindestens eine Stickstoff-haltige Verbindung V, vorzugsweise ausgewählt aus der Gruppe bestehend aus 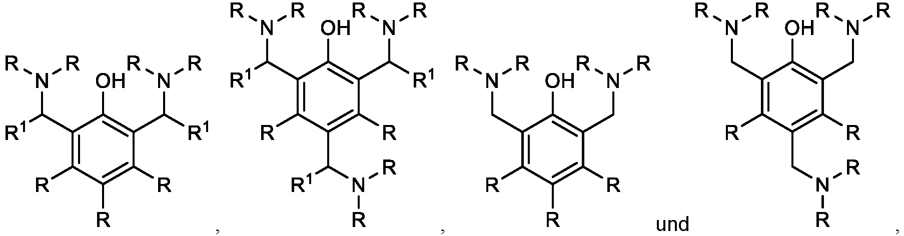 wobei
- R =: jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann, optional enthaltend Heteroatome wie O oder N,
- R¹ =: jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann.

Entsprechende Stickstoff-haltige Verbindungen V sind an sich bekannt und können z.B. durch Umsetzung von ggf. modifizierten Phenolen mit Aldehyden und Aminen erhalten werden.

Vorzugsweise kann die mindestens eine Stickstoff-haltige Verbindung V durch Umsetzung von Phenol mit Aldehyd, vorzugsweise Aldehyd mit 1 bis 20 Kohlenstoffen, bevorzugt Formaldehyd, Acetaldehyd, Propionaldehyd, Butyraldehyde, Benzaldehyd, Zimtaldehyd, Trimethylhexanal, Pelargonaldehyd, Acrolein und/oder Furfural und Amin im Sinne der Mannich-Reaktion hergestellt werden, wobei als Ausgangsmaterial auch modifizierte Phenole verwendet werden können, die mindestens einen Substituenten am aromatischen Kern tragen, wie vorzugsweise Kresol, Xylenole, Propylphenole, Styrylphenole, Alkylphenole und/oder Cardanol, ebenso können vorzugsweise auch Resorcin und/oder Catechol-basierende Strukturen als modifizierte Phenole eingesetzt werden, bevorzugt kann die mindestens eine Stickstoff-haltige Verbindung V durch Umsetzung von Phenol mit Formaldehyd und mindestens einem primären oder sekundären Aminen, vorzugsweise ausgewählt aus der Gruppe bestehend aus Dimethylamin, Diethylamin, Dipropylamin, Diisopropylamin, Dibutylamin, Ethanolamin, Diethanolamin, Trimethylethandiamin, Isophorondiamin, Dimethylpropylamin, Diethylentriamin, Triethylentetramin, Methylhydroxyethylamin, Dimethylaminopropylamin, Pyrrolidin, Pyrrol, Morpholin, Piperazin, N,N-dimethyl-2-(2-methylamino-ethoxy)ethan-1-amin, N-[2-[2-(dimethylamino)ethoxy]ethyl]-N-methyl-1,3-propanediamine, Diisopropyldimethyldiethylentriamin, Bis-(dimethylaminopropyl)amine Trimethylaminoethylethanolamin, N,N'-diisopropyl-N-methyl-bis (aminoethyl) ether und N,N'-Diisobutyl-N-methyl-bis (aminoethyl) ether hergestellt werden, wobei als Ausgangsmaterial wiederum auch modifizierte Phenole verwendet werden können.

Es ist besonders bevorzugt, wenn die mindestens eine Stickstoff-haltige Verbindung V ausgewählt ist aus der Gruppe bestehend aus 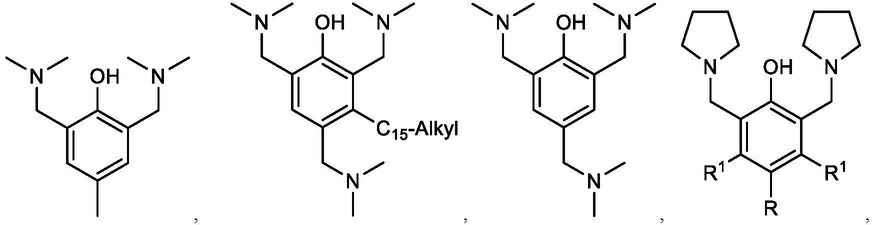 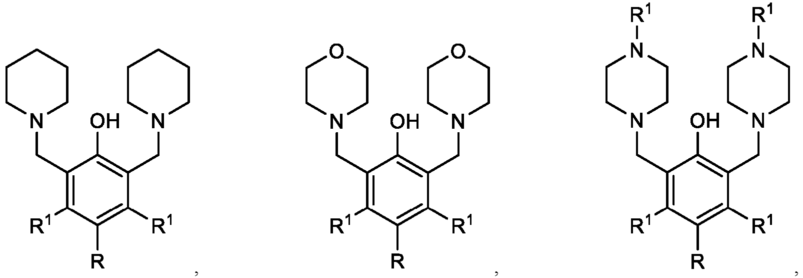 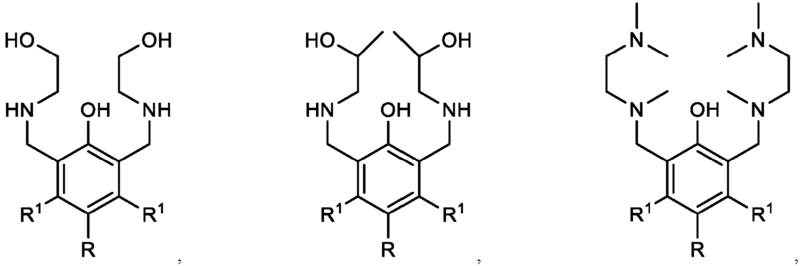 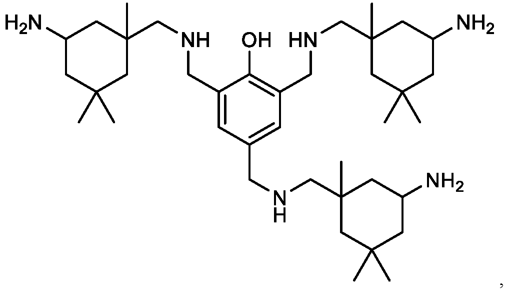 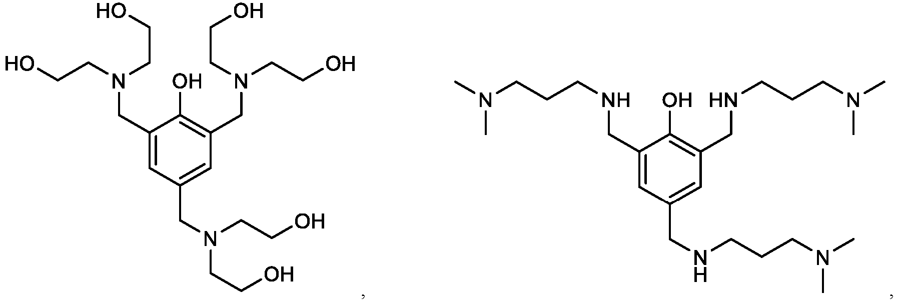 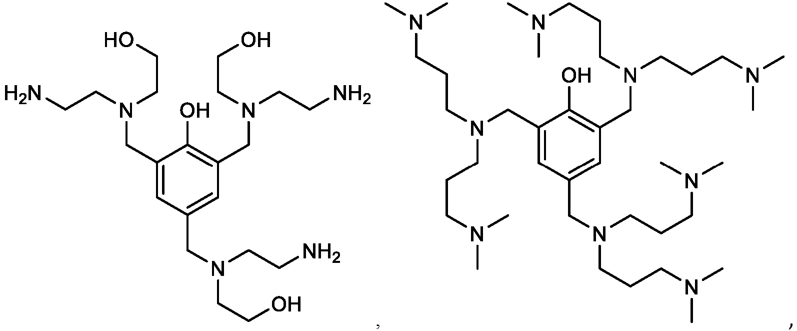 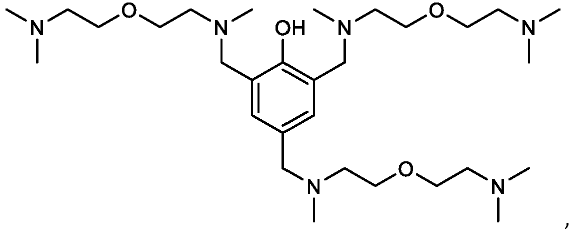 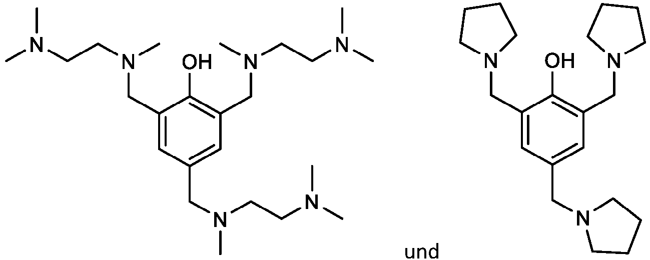 wobei
- R =: jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann, optional enthaltend Heteroatome wie O oder N,
- R¹ =: jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann,
wobei der Einsatz von 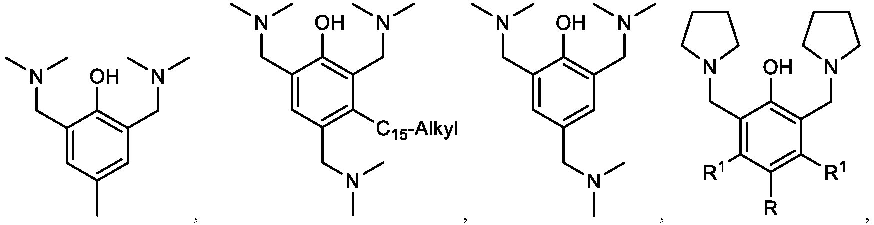 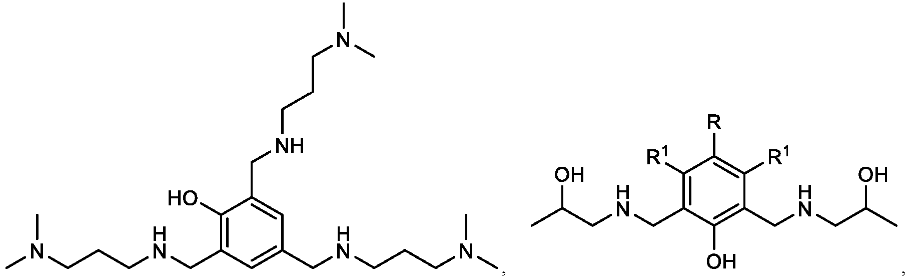 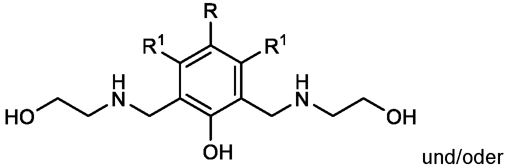 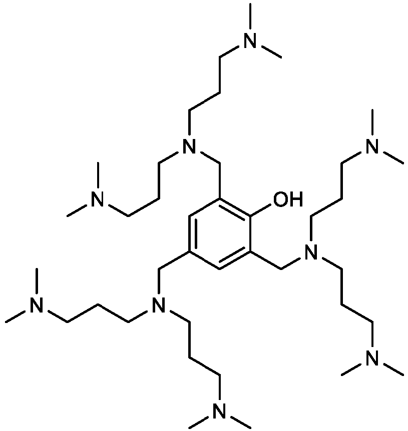 wobei
- R =: jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann, optional enthaltend Heteroatome wie O oder N,
- R¹ =: jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann,
ganz besonders bevorzugt ist.

Am meisten bevorzugt ist der Einsatz von 2,6-Bis[(dimethylamino)methyl]-4-methyl-phenol, 2,4,6-Tris[(dimethylamino)methyl]cardanol, 2,4,6-Tris[(dimethylamino)methyl]phenol, 2,6-Bis[(dimethyl-amino)methyl]-cardanol, 2,4,6-Tris[(hydroxyethylamino)methyl]phenol, 2,4,6-Tris[(hydroxypropyl-amino)methyl]phenol und/oder 2,4,6-Tris[(dimethylaminopropylamino)methyl]phenol.

Die mindestens eine Stickstoff-haltige Verbindung V ist vorzugsweise in einer Gesamtmenge von 1 bis 80 Gew.-%, bevorzugt 2 bis 60 Gew.-%, in der erfindungsgemäßen Zusammensetzung enthalten, Gew.-% jeweils bezogen auf die gesamte erfindungsgemäße Zusammensetzung.

Es ist bevorzugt, wenn in der erfindungsgemäßen Zusammensetzung insgesamt enthaltenes Zink(II)-Carboxylat und insgesamt enthaltene Sticktoff-haltige Verbindung V im Mengenverhältnis von 1 zu 0,5 bis 1 zu 5 Gewichtsteilen zueinander vorliegen.

Im Sinne der vorliegenden Erfindung ist es ganz besonders bevorzugt, das mindestens ein Zink(II)-Carboxylat, zur Verwendung in PU- oder PIR-Reaktionsmischungen unter Einsatz mindestens eines Trägermediums in gelöster Form (bzw. flüssiger Form bei 25°C und Normaldruck) einzubringen.

Vorzugsweise kann auch eine Kombination aus mindestens einem Zink(II)-Carboxylat und mindestens einer Stickstoff-haltigen Verbindung V gewählt werden, um das Einbringen in gelöster Form zu gewährleisten, wobei diese Kombination an sich vorzugsweise bereits eine ausreichend niedrige Viskosität und auch keine Neigung zur Kristallisation aufweisen kann.

Die Verwendung eines Trägermediums ist optional. Jedoch ist es bevorzugt, das erfindungsgemäße mindestens eine Zink(II)-Carboxylat in mindestens einem Trägermedium dem Reaktionsgemisch zuzugeben.

Der Einsatz von Trägermedien ist zwar optional, er kann aber vorteilhaft und bevorzugt sein, weil er dazu beitragen kann, dass z.B. gewünschte Viskositäten eingestellt werden können und/oder das beispielsweise etwaige Kristallisationsneigungen verringert werden können.

Es ist besonders bevorzugt, wenn in der erfindungsgemäßen Zusammensetzung überdies mindestens ein zusätzlicher Trimerisierungskatalysator enthalten ist, vorzugsweise ausgewählt aus Carboxylaten von Ammonium, Kalium und/oder anderen Alkali- oder Erdalkalimetallen,
bevorzugt ausgewählt aus Kalium-Carboxylaten und Carboxylaten von Ammonium-Kationen, ganz besonders bevorzugt ausgewählt aus der Gruppe bestehend aus Kaliumacetat, Kaliumformiat, Kaliumpropionat, Kaliumbutanoat, Kaliumpentanoat, Kaliumhexanoat, Kaliumheptanoat, Kalium-2-ethylhexanoat, Kaliumpivalat, Kaliumoctoat, Kaliumbutyrat, Kaliumisobutyrat, Kalium nonanoat, Kaliumdecanoat, Kaliumrizinoleat, Kaliumstearat, Kaliumneodecanoat und Carboxylaten von Tetramethylammonium, Tetraethylammonium, Triethylmethylammonium, Tetrapropylammonium, Tetrabutylammonium, Dimethyldiallylammonium, Trimethyl-(2-hydroxypropyl)ammonium, Triethyl-(2-hydroxypropyl)ammonium, Tripropyl-(2-hydroxypropyl)ammonium, Tributyl-(2-hydroxypropyl)-ammonium, Trimethyl-(2-hydroxyethyl)ammonium, Triethyl-(2-hydroxyethyl)ammonium, Tripropyl-(2-hydroxyethyl)ammonium, Tributyl-(2-hydroxyethyl)ammonium, Dimethylbenzyl-(2-hydroxyethyl)-ammonium und/oder Dimethylbenzyl-(2-hydroxypropyl)ammonium, wobei die Carboxylate vorzugweise Acetate, Propionate, Butanoate, Pentanoate Pivalate, Octoate, Nonanoate, Decanoate, Neodecanoate, Rizinoleate und/oder Stearate sind.

Der oder die zusätzlichen Trimerisierungskatalysatoren als solche enthalten selbst kein Zink.

Mit dem mindestens einen zusätzlichen Trimerisierungskatalysator kann die Reaktionsgeschwindigkeit gewünschtenfalls auf das bevorzugte Maß angepasst werden. Der mindestens eine zusätzliche Trimerisierungskatalysator kann auch Bestandteil einer Zink(II)-Carboxylat-haltigen Zubereitung sein. Er kann der erfindungsgemäßen Zusammensetzung aber auch separat, also getrennt von Zink(II)-Carboxylat(en), zugeführt werden.

Eine bevorzugte erfindungsgemäße Zusammensetzung umfasst den mindestens einen zusätzlichen Trimerisierungskatalysator in einer Gesamtmenge von 10 bis 90 Gew.-%, vorzugsweise von 20 bis 80 Gew.-%, bezogen auf die gesamte erfindungsgemäße Zusammensetzung.

Es ist bevorzugt, dass optional (vorzugsweise obligatorisch) enthaltener, zusätzlicher Trimerisierungskatalysator getrennt von dem mindestens einen Zink(II)-Carboxylat in die erfindungsgemäße Zusammensetzung eingebracht wird.

Weiterhin ist es bevorzugt, wenn die erfindungsgemäß Zusammensetzung frei ist von Sb-Carboxylat und/oder Sn-Carboxylat.

Die erfindungsgemäße Zusammensetzung enthält, wie oben bereits gesagt, mindestens ein tertiäres Amin der Formel (X) 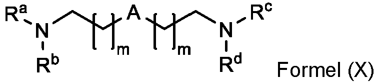 mit
- m: jeweils unabhängig voneinander gleich 1 oder 2,
- A: gleich O, S oder N-R^{e},
- R^{a}, R^{b}, R^{c}, R^{d} und R^{e},: jeweils unabhängig voneinander, gleiche oder verschiedene lineare, verzweigte oder cyclische Alkylreste mit 1 bis 20 Kohlenstoffen.

Es ist besonders bevorzugt, wenn das mindestens eine tertiäre Amin der Formel (X) ausgewählt ist aus der Gruppe bestehend aus Pentamethyldiethylentriamin, Bis-(2-dimethylaminoethyl)ether, Tris-(dimethylaminopropyl)amin, N-[2-[2-(dimethylamino)ethoxy]ethyl]-N-methyl-1,3-propanediamin, Diisopropyltrimethyldiethylentriamin, Bis-(dimethylaminopropyl)methylamin, Trimethylaminoethylethanolamin, Bis-(2-isopropylmethylaminoethyl)ether, Bis-(2-isobutylmethylaminoethyl)ether, N,N'-diisopropyl-N,N`-dimethyl-bis (aminoethyl) ether, N,N,N'-triisopropyl-N'-methyl-bis (aminoethyl) ether und N,N'-diisobutyl-N,N'-dimethyl-bis (aminoethyl) ether.

Das mindestens eine tertiäre Amin der Formel (X), vorzugsweise Pentamethyldiethylentriamin, Bis-(2-dimethylaminoethyl)ether, Tris(dimethylaminopropyl)amine, N-[2-[2-(dimethylamino)ethoxy]ethyl]-N-methyl-1,3-propanediamine, 2-[[2-[2-(Dimethylamino)ethoxy]ethyl]methylamino]ethanol, Diisopropyltrimethyldiethylentriamin, Bis-(dimethylaminopropyl)methylamine und/oder Trimethylaminoethylethanolamin, hat insbesondere die Funktion als Katalysator zu wirken.

Eine bevorzugte erfindungsgemäße Zusammensetzung umfasst mindestens ein tertiäres Amin der Formel (X), vorzugsweise Pentamethyldiethylentriamin, Bis-(2-dimethylaminoethyl)ether, Tris(dimethylaminopropyl)amine, N-[2-[2-(dimethylamino)ethoxy]ethyl]-N-methyl-1,3-propanediamine, 2-[[2-[2-(Dimethylamino)ethoxy]ethyl]methylamino]ethanol, Diisopropyltrimethyldiethylentriamin, Bis-(dimethylaminopropyl)methylamine und/oder Trimethylaminoethylethanolamin, in einer Gesamtmenge von > 0 bis 20 Gew.-%, vorzugsweise von 1 bis 15 Gew.-%, bezogen auf die gesamte erfindungsgemäße Zusammensetzung.

Eine besonders bevorzugte erfindungsgemäße Zusammensetzung umfasst also eine Polyisocyanat-Komponente, eine Polyolkomponente, mindestens ein Zink(II)-Carboxylat, mindestens ein tertiäres Amin der Formel (X), mindestens eine Stickstoff-haltige Verbindung V und mindestens einen zusätzlichen Trimerisierungskatalysator.

Es entspricht einer bevorzugten Ausführungsform der Erfindung, wenn der gesamte Massenanteil an insgesamt eingesetztem Zink(II)-Carboxylat am fertigen Polyurethan- oder Polyisocyanurat-Schaum vorzugsweise von 0,01 bis 10 Gew.-%, bevorzugt von 0,1 bis 5 Gew.-% beträgt.

Es ist bevorzugt, wenn die erfindungsgemäße Zusammensetzung Wasser und/oder mindestens ein Treibmittel, optional mindestens ein Flammschutzmittel und/oder weitere Additive umfasst, welche bei der Herstellung von Polyurethan- oder Polyisocyanurat-Schaumstoff vorteilhaft einsetzbar sind. Es können neben dem Zink(II)-Carboxylat auch noch weitere Katalysatoren enthalten sein.

Eine besonders bevorzugte erfindungsgemäße Zusammensetzung enthält die folgenden Bestandteile:
a) eine Polyisocyanat-Komponente,
b) eine Polyolkomponente,
c) mindestens ein Zink(II)-Carboxylat, mit der Maßgabe, dass das enthaltene Zink(II)-Carboxylat in stöchiometrischer Form eingesetzt wird, also mit Zn(II) und Carboxylat im molaren Verhältnis von 1 zu 2,
d) mindestens ein tertiäres Amin der Formel (X) 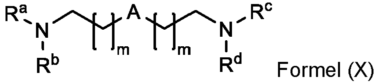 mit
   - m: jeweils unabhängig voneinander gleich 1 oder 2,
   - A: gleich O, S oder N-R^{e},
   - R^{a}, R^{b}, R^{c}, R^{d} und R^{e},: jeweils unabhängig voneinander, gleiche oder verschiedene lineare, verzweigte oder cyclische Alkylreste mit 1 bis 20 Kohlenstoffen
e) mindestens einen Schaumstabilisator,
f) mindestens ein Treibmittel,
g) mindestens eine Stickstoff-haltige Verbindung V,
h) mindestens ein zusätzlicher Trimerisierungskatalysator,
i) optional weitere Additive, vorzugsweise Füllstoffe und/oder Flammschutzmittel.

Wie bereits ersichtlich wurde, kann das mindestens eine Zink(II)-Carboxylat auf unterschiedliche Weise in die erfindungsgemäße Zusammensetzung eingebracht werden, z.B. pur, gelöst oder gemischt mit anderen Bestandteilen.

Es ist besonders bevorzugt, das mindestens eine Zink(II)-Carboxylat nicht pur, sondern in Gestalt einer Zink(II)-Carboxylat-haltigen Zubereitung in die erfindungsgemäße Zusammensetzung einzubringen, wobei die Zubereitung vorzugsweise zusätzlich mindestens eine Stickstoff-haltige Verbindung V umfasst sowie optional mindestens ein Trägermedium enthalten kann.

Ein weiterer Gegenstand der Erfindung ist daher eine Zink(II)-Carboxylat-haltige Zubereitung, geeignet zur Katalyse bei der Herstellung von PU- oder PIR-Schaumstoff, welche umfasst:
i) mindestens ein Zink(II)-Carboxylat, in Gesamtmenge von 2 bis 50 Gew.-%, vorzugsweise 5 bis 45 Gew.-%, besonders bevorzugt 10 bis 40 Gew%, mit der Maßgabe, dass das enthaltene Zink(II)-Carboxylat in stöchiometrischer Form eingesetzt wird, also mit Zn(II) und Carboxylat im molaren Verhältnis von 1 zu 2,
ii) optional mindestens ein Trägermedium, in Gesamtmenge von 0 bis 95 Gew.-%, vorzugsweise 10 bis 90 Gew.-%, besonders bevorzugt 20 bis 70 Gew%,
iii) mindestens eine Stickstoff-haltige Verbindung V, welche mindestens zwei N-Atome aufweist und bei der es sich um ein modifiziertes Phenol handelt, in Gesamtmenge von 1 bis 90 Gew.- %, vorzugsweise 2 bis 60 Gew.-%, besonders bevorzugt 5 bis 50 % Gew-%,
   Gew.-% jeweils bezogen auf die gesamte Zubereitung, wobei die Komponenten (i) bis (iii) zusammen vorzugsweise mindestens 51 Gew.-%, bevorzugt mindestens 70 Gew.-%, besonders bevorzugt mindestens 80 Gew.-% der gesamten Zubereitung ausmachen müssen, sowie bevorzugt zusätzlich umfassend
iv) mindestens ein tertiäres Amin, wie in Anspruch 1 oder 7 definiert, in Mengen von 1 bis 30 Gew-%, vorzugsweise 2 bis 25 Gew-%, besonders bevorzugt 5 bis 20 Gew-%, bezogen auf die gesamte Zubereitung,
v) optional mindestens einen zusätzlichen Trimerisierungskatalysator, vorzugsweise wie in Anspruch 8 definiert, in Mengen von 1 bis 90 Gew.-%, vorzugsweise 2 bis 60 Gew.-%, besonders bevorzugt 5 bis 50 % Gew-%, Gew.-% wiederum jeweils bezogen auf die gesamte Zubereitung,
   wobei die Komponenten i) bis v) zusammen vorzugsweise mindestens 52 Gew.-% der gesamten Zubereitung ausmachen müssen.

Die erfindungsgemäßen Zink-haltigen Zubereitungen ermöglichen eine höhere Zink-Konzentration und/oder eine niedrigere Viskosität als nach dem Stand der Technik bekannt, bezogen auf die jeweilige Zubereitung

Es ist bevorzugt, dass die Zink(II)-Carboxylat-haltige Zubereitung keinen zusätzlichen Trimerisierungskatalysator umfasst. In einer dazu alternativen Ausführungsform kann es bevorzugt sein, dass die Zink(II)-Carboxylat-haltige Zubereitung als weiteren Bestandteil v) mindestens einen zusätzlichen Trimerisierungskatalysator umfasst, vorzugsweise wie in Anspruch 8 definiert, bevorzugt in Mengen von 1 bis 90 Gew.-%, weiter bevorzugt 2 bis 60 Gew.-%, besonders bevorzugt 5 bis 50 % Gew-%, Gew.-% wiederum jeweils bezogen auf die gesamte Zubereitung,
wobei die Komponenten i) bis v) zusammen vorzugsweise mindestens 52 Gew.-%, bevorzugt mindestens 80 Gew.-%, besonders bevorzugt mindestens 90 Gew.-% der gesamten Zubereitung ausmachen müssen.

Es ist bevorzugt, mindestens ein Trägermedium einzusetzen, um eine bevorzugte Zink(II)-Carboxylat-haltige Zubereitung zur Verfügung zu stellen, die besonders unkompliziert eingesetzt werden kann. Bevorzugt wäre hier z.B. eine Mischung oder Lösung, die wenig oder keine Trübung aufweist, also vorzugsweise keine Emulsionen, Suspensionen oder Dispersionen, bei denen es zu Problemen wie beispielsweise Phasentrennung oder Sedimentation kommen kann.

Ebenso wäre eine möglichst niedrige Viskosität bevorzugt, so dass die Zubereitung z.B. keine speziellen Anforderungen an Pumpen oder andere technische Geräte zur Verarbeitung stellt. Bevorzugte Viskositäten sind kleiner 10 Pa·s, bevorzugt kleiner 8 Pa·s, besonders bevorzugt kleiner 6 Pa·s, gemessen mit einem Brookfield Viskosimeter bei 25°C.

Ein weiterer Gegenstand der Erfindung ist ein Verfahren zur Herstellung von Polyurethan- oder Polyisocyanurat-Schaumstoff, durch Umsetzung einer Polyolkomponente mit einer Polyisocyanatkomponente, dadurch gekennzeichnet, dass es unter Einsatz einer erfindungsgemäßen Zusammensetzung wie zuvor beschrieben, bevorzugt nach einem der Ansprüche 1 bis 10, oder unter Einsatz einer erfindungsgemäßen Zink(II)-Carboxylat-haltigen Zubereitung, vorzugsweise nach Anspruch 11, erfolgt,
wobei es bei Einsatz einer Zink(II)-Carboxylat-haltigen Zubereitung bevorzugt ist, dass der gesamte Massenanteil an Zink(II)-Carboxylat-haltige Zubereitung am fertigen Polyurethanschaumstoff 0,05 bis 10 Gew.-%, bevorzugt von 0,2 bis 5 Gew.-% beträgt.

Dabei können neben der bevorzugt einsetzbaren erfindungsgemäßen Zink(II)-Carboxylat-haltigen Zubereitung auch noch weitere Katalysatoren eingesetzt werden, ganz besonders bevorzugt mindestens ein tertiäres Amin der Formel (X), wie zuvor beschrieben.. Ebenfalls bevorzugt ist der zusätzliche Einsatz mindestens eines Trimerisierungskatalysators, wie zuvor beschrieben.

Dabei ist es bevorzugt, dass die Zink(II)-Carboxylat-haltige Zubereitung mindestens ein Trägermedium umfasst, besonders bevorzugt wird die Zink(II)-Carboxylat-haltige Zubereitung dem Reaktionsgemisch zur Herstellung des Polyurethan- oder Polyisocyanurat-Schaumstoffs, vorzugsweise PU- oder PIR-Hartschaumstoffs, in einem Trägermedium zugeführt, vorzugsweise umfassend Glykole, Alkoxylate und/oder Öle synthetischer und/oder natürlicher Herkunft.

Ein weiterer Gegenstand der Erfindung ist ein Polyurethan- oder Polyisocyanurat-Schaumstoff, vorzugsweise Polyurethan- oder Polyisocyanurat-Hartschaumstoff erhältlich durch das erfindungsgemäße Verfahren, wie zuvor beschrieben.

Ein weiterer Gegenstand der Erfindung ist die Verwendung einer Zusammensetzung nach einem der Ansprüche 1 bis 10 oder einer Zubereitung gemäß Anspruch 11 bei der Herstellung von Polyurethan- oder Polyisocyanurat-Schaumstoffen, vorzugsweise Polyurethan- oder Polyisocyanurat-Hartschaumstoffen, bevorzugt zur Verbesserung der Gebrauchseigenschaften des Polyurethan- oder Polyisocyanurat-Schaumstoffs, vorzugsweise Polyurethan- oder Polyisocyanurat-Hartschaumstoffs, insbesondere zur Erhöhung der Stauchhärte des Polyurethan- oder Polyisocyanurat-Schaumstoffs, vorzugsweise Polyurethan- oder Polyisocyanurat-Hartschaumstoffs zu einem frühen Zeitpunkt, im Vergleich zu Polyurethan- oder Polyisocyanurat-Schaumstoffen, vorzugsweise Polyurethan- oder Polyisocyanurat-Hartschaumstoffen, die ohne Zink(II)-Carboxylat hergestellt wurden, Stauchhärte bestimmbar nach DIN EN ISO 844:2014-11. Dabei ist unter dem Ausdruck "früher Zeitpunkt" vorzugsweise ein Zeitpunkt im Bereich von 3 Minuten bis 10:30 Minuten, bevorzugt 8 Minuten, nach Start der Polymerisation, vorzugsweise nach Start der Polymerisation durch Zugabe der Polyisocyanat-Komponente, zu verstehen.

Außerdem ist ein weiterer Gegenstand der vorliegenden Erfindung die Verwendung von erfindungsgemäßen Polyurethan- oder Polyisocyanurat-Schaumstoffen, vorzugsweise Polyurethan- oder Polyisocyanurat-Hartschaumstoffen für Zwecke der thermischen Isolation, vorzugsweise als Dämmplatten und/oder Isolationsmittel sowie für Kühlapparaturen, die als Isoliermaterial einen erfindungsgemäßen Polyurethan- oder Polyisocyanurat-Schaumstoff, vorzugsweise Polyurethan- oder Polyisocyanurat-Hartschaumstoff aufweisen.

Nachfolgend werden einzelne, bevorzugt einsetzbare Komponenten noch genauer beschrieben.

Als Polyolkomponente geeignete Polyole im Sinne der vorliegenden Erfindung sind alle organischen Verbindungen mit mindestens zwei gegenüber Isocyanaten reaktiven Gruppen, vorzugsweise OH-Gruppen, sowie deren Zubereitungen. Insbesondere umfasst die Polyolkomponente mindestens eine organische Verbindung, die mindestens zwei Hydroxylgruppen (-OH) enthält. Vorzugsweise können Mischungen aus mindestens zwei geeigneten Polyolen eingesetzt werden.

Bevorzugte Polyole sind alle zur Herstellung von Polyurethan-Systemen, insbesondere Polyurethan-Beschichtungen, Polyurethan-Elastomeren oder auch Schaumstoffen üblicherweise einsetzbaren Polyetherpolyole und/oder Polyesterpolyole und/oder hydroxylgruppenhaltigen aliphatischen Polycarbonate, insbesondere Polyetherpolycarbonatpolyole und/oder Polyole natürlicher Herkunft, sogenannte "natural oil based polyols" (NOPs). Vorzugsweise besitzen die Polyole eine Funktionalität von 1.8 bis 8 und zahlengemittelte Molekulargewichte vorzugsweise im Bereich von 500 bis 15000. Vorzugsweise kommen die Polyole mit OH-Zahlen im Bereich von 10 bis 1200 mg KOH/g zum Einsatz.

Einsetzbar sind vorzugsweise Polyetherpolyole. Diese können nach bekannten Verfahren hergestellt werden, beispielsweise durch anionische Polymerisation von Alkylenoxiden in Gegenwart von Alkalihydroxiden, Alkalialkoholaten oder Aminen als Katalysatoren und unter Zusatz mindestens eines Startermoleküls, dass bevorzugt 2 oder 3 reaktive Wasserstoffatome gebunden enthält oder durch kationische Polymerisation von Alkylenoxiden in Gegenwart von Lewis-Säuren wie beispielsweise Antimonpentachlorid oder Bortrifluorid-Etherat oder durch Doppelmetallcyanidkatalyse. Geeignete Alkylenoxide enthalten 2 bis 4 Kohlenstoffatome im Alkylenrest. Beispiele sind Tetrahydrofuran, 1,3-Propylenoxid, 1,2- bzw. 2,3-Butylenoxid; vorzugsweise werden Ethylenoxid und 1,2-Propylenoxid eingesetzt. Die Alkylenoxide können einzeln, kumulativ, blockweise, alternierend nacheinander oder als Mischungen verwendet werden. Als Startmoleküle kommen insbesondere Verbindungen mit mindestens 2, vorzugsweise 2 bis 8 Hydroxylgruppen oder mit mindestens zwei primären Aminogruppen im Molekül zum Einsatz. Als Startermoleküle eingesetzt werden können z.B. Wasser, 2-, 3- oder 4-wertige Alkohole wie Ethylenglykol, Propandiol-1,2 und -1,3, Diethylenglykol, Dipropylenglykol, Glycerin, Trimethylolpropan, Pentaerythrit, Rizinusöl usw., höhere polyfunktionelle Polyole, insbesondere Zuckerverbindungen wie beispielsweise Glucose, Sorbit, Mannit und Saccharose, mehrwertige Phenole, Resole, wie z.B. oligomere Kondensationsprodukte aus Phenol und Formaldehyd und Mannich-Kondensate aus Phenolen, Formaldehyd und Dialkanolaminen sowie Melamin, oder Amine wie Anilin, EDA, TDA, MDA und PMDA, besonders bevorzugt TDA und PMDA. Die Wahl des geeigneten Startermoleküls ist abhängig von dem jeweiligen Anwendungsgebiet des resultierenden Polyetherpolyols bei der Polyurethanherstellung

Einsetzbar sind vorzugsweise Polyesterpolyole. Diese basieren auf Estern mehrwertiger aliphatischer oder aromatischer Carbonsäuren, bevorzugt mit 2 bis 12 Kohlenstoffatomen. Beispiele für aliphatische Carbonsäuren sind Bernsteinsäure, Glutarsäure, Adipinsäure, Korksäure, Azelainsäure, Sebacinsäure, Decandicarbonsäure, Maleinsäure und Fumarsäure. Beispiele für aromatische Carbonsäuren sind Phthalsäure, Isophthalsäure, Terephthalsäure und die isomeren Naphthalindicarbonsäuren. Die Polyesterpolyole werden durch Kondensation dieser mehrwertigen Carbonsäuren mit mehrwertigen Alkoholen, vorzugsweise von Diolen oder Triolen mit 2 bis 12, besonders bevorzugt mit 2 bis 6 Kohlenstoffatomen, bevorzugt Trimethylolpropan und Glycerin erhalten.

Einsetzbar sind vorzugsweise Polyetherpolycarbonatpolyole. Dies sind Polyole, welche Kohlenstoffdioxid als Carbonat gebunden enthalten. Da Kohlenstoffdioxid bei vielen Prozessen in der chemischen Industrie in großen Mengen als Nebenprodukt entsteht, ist die Verwendung von Kohlendioxid als Comonomer in Alkylenoxid-Polymerisationen aus kommerzieller Sicht von besonderem Interesse. Ein teilweiser Ersatz von Alkylenoxiden in Polyolen durch Kohlendioxid hat das Potential, die Kosten für die Herstellung von Polyolen deutlich zu senken. Außerdem ist die Verwendung von CO₂ als Comonomer ökologisch sehr vorteilhaft, da diese Reaktion die Umsetzung eines Treibhausgases zu einem Polymer darstellt. Die Herstellung von Polyetherpolycarbonatpolyolen durch Anlagerung von Alkylenoxiden und Kohlendioxid an H-funktionelle Startsubstanzen unter Verwendung von Katalysatoren ist seit langem bekannt. Verschiedene Katalysatorsysteme können hierbei zum Einsatz kommen: Die erste Generation stellten heterogene Zink- oder Aluminiumsalze dar, wie sie beispielsweise in US-A 3900424 oder US-A 3953383 beschrieben sind. Des Weiteren sind mono- und binukleare Metallkomplexe zur Copolymerisation von CO2 und Alkylenoxiden erfolgreich eingesetzt worden (vgl. z.B. WO 2010/028362, WO 2009/130470, WO 2013/022932 oder WO 2011/163133). Die wichtigste Klasse von Katalysatorsystemen für die Copolymerisation von Kohlenstoffdioxid und Alkylenoxiden stellen die Doppelmetallcyanidkatalysatoren, auch als DMC-Katalysatoren bezeichnet, dar (vgl. z.B. US-A 4500704, WO 2008/058913). Geeignete Alkylenoxide und H-funktionelle Startsubstanzen sind solche, die auch zur Herstellung von carbonatfreien Polyetherpolyolen - wie oben beschrieben - eingesetzt werden.

Einsetzbar sind vorzugsweise Polyole auf Basis nachwachsender Rohstoffe "Natural oil based polyols" (NOPs). NOPs zur Herstellung von Polyurethanschäumen sind mit Blick auf die langfristig begrenzte Verfügbarkeit fossiler Ressourcen, namentlich Öl, Kohle und Gas, und vor dem Hintergrund steigender Rohölpreise von zunehmendem Interesse und bereits vielfach in solchen Anwendungen beschrieben (vgl. z.B. WO 2005/033167; US 2006/0293400, WO 2006/094227, WO 2004/096882, US 2002/0103091, WO 2006/116456 und EP 1678232). Mittlerweile sind auf dem Markt eine Reihe dieser Polyole von verschiedenen Herstellern verfügbar (vgl. z.B. WO2004/020497, US2006/0229375, WO2009/058367). In Abhängigkeit vom Basis-Rohstoff (z.B. Sojabohnenöl, Palmöl oder Rizinusöl) und die daran angeschlossene Aufarbeitung ergeben sich Polyole mit unterschiedlichem Eigenschaftsbild. Hierbei können im Wesentlichen zwei Gruppen unterschieden werden: a) Polyole auf Basis nachwachsender Rohstoffe, die soweit modifiziert werden, dass sie zu 100 % zur Herstellung von Polyurethanen eingesetzt werden können (vgl. z.B. WO2004/020497, US2006/0229375); b) Polyole auf Basis nachwachsender Rohstoffe, die bedingt durch ihre Aufarbeitung und Eigenschaften nur zu einem gewissen Anteil das petrochemisch basierte Polyol ersetzen können (vgl. z.B. WO2009/058367).

Eine weitere Klasse von vorzugsweise einsetzbaren Polyolen stellen die sogenannten Füllkörperpolyole (Polymerpolyole) dar. Diese zeichnen sich dadurch aus, dass sie feste organische Füllstoffe vorzugsweise bis zu einem Feststoffgehalt von 40 % oder mehr in disperser Verteilung enthalten. Einsetzbar sind unter anderem SAN-, PHD- und PIPA-Polyole. SAN-Polyole sind hochreaktive Polyole, welche ein Copolymer auf der Basis von Styrol/Acrylnitril (SAN) dispergiert enthalten. PHD-Polyole sind hochreaktive Polyole, welche Polyharnstoff ebenfalls in dispergierter Form enthalten. PIPA-Polyole sind hochreaktive Polyole, welche ein Polyurethan, beispielsweise durch in situ-Reaktion eines Isocyanats mit einem Alkanolamin in einem konventionellen Polyol gebildet, in dispergierter Form enthalten.

Bevorzugt können Polyole zum Einsatz kommen, die eine Molmasse kleiner als 1000g/mol haben. Weiter bevorzugt sind Polyole mit einer Funktionalität kleiner 3. Insbesondere ist es bevorzugt keine Triole mit Molmassen über 1000g/mol einzusetzen. Dies entspricht jeweils einer besonders bevorzugten Form der Erfindung.

Ein bevorzugtes Verhältnis von Isocyanat und Polyol, ausgedrückt als Index der Formulierung, d.h. als stöchiometrisches Verhältnis von Isocyanat-Gruppen zu gegenüber Isocyanat reaktiven Gruppen (z.B. OH-Gruppen, NH-Gruppen) multipliziert mit 100, liegt im Bereich von 10 bis 1000, bevorzugt 40 bis 700, bevorzugter 60 bis 600, weiter bevorzugt 150 bis 550, noch weiter bevorzugt 250 bis 500, ganz besonders bevorzugt 300 bis 450. Ein Index von 100 steht für ein molares Verhältnis der reaktiven Gruppen von 1 zu 1.

Bevorzugt sind PIR-Formulierungen auf Basis von mind. 70, 80 oder 90 Gew.-% Polyester in der Polyol-Komponente.

In einer besonders bevorzugten Ausführungsform werden Polyesterpolyole auf Basis von aromatischen Carbonsäuren in mehr als 50 Massenteile, bevorzugt mehr als 70 Massenteile eingesetzt, bezogen auf 100 Massenteile der gesamten Polyolkomponente.

Bevorzugte aromatische Polyesterpolyol haben OH-Zahlen im Bereich von 150 bis 400 mg KOH/g, bevorzugt 170 bis 350, ganz besonders bevorzugt 180 bis 300 mg KOH/g

Als Polyisocyanat-Komponente wird vorzugsweise mindestens ein organisches Polyisocyanat mit mindestens zwei Isocyanat-Funktionen eingesetzt.

Geeignete Polyisocyanate im Sinne dieser Erfindung sind alle Isocyanate, die mindestens zwei Isocyanat-Gruppen enthalten. Vorzugsweise können alle an sich bekannten aliphatischen, cycloaliphatischen, arylaliphatischen und vorzugsweise aromatischen mehrfunktionalen Isocyanate verwendet werden. Besonders bevorzugt werden Isocyanate in einem Bereich von 60 bis 200 mol% relativ zu der Summe der isocyanat-verbrauchenden Komponenten eingesetzt.

Vorzugsweise können Mischungen aus mindestens zwei geeigneten Polyisocyanaten eingesetzt werden.

Beispielhaft genannt werden können hier Alkylendiisocyanate mit 4 bis 12 Kohlenstoffatomen im Alkylenrest, wie 1,12-Dodecandiisocyanat, 2-Ethyltetramethylendiisocyanat-1,4, 2-Methylpentamethylen-diisocyanat-1,5, Tetramethylendiisocyanat-1,4, und vorzugsweise Hexamethylendiisocyanat-1,6 (HMDI), cycloaliphatische Diisocyanate, wie Cyclohexan-1,3- und 1-4-diisocyanat sowie beliebige Gemische dieser Isomeren, 1-Isocyanato-3,35-trimethyl-5-isocyanatomethylcyclohexan (Isophorondiisocyanat oder kurz IPDI), 2,4- und 2,6-Hexahydrotoluylendiisocyanat sowie die entsprechenden Isomerengemische, und vorzugsweise aromatische Di- und Polyisocyanate, wie beispielsweise 2,4- und 2,6-Toluoldiisocyanat (TDI) und die entsprechenden Isomerengemische, Naphthalindiisocyanat, Diethyltoluoldiisocyanat, Mischungen aus 2,4'- und 2,2`-Diphenylmethandiisocyanaten (MDI) und Polyphenylpolymethylenpolyisocyanate (Roh-MDI) und Mischungen aus Roh-MDI und Toluoldiisocyanaten (TDI). Die organischen Di- und Polyisocyanate können einzeln oder in Form ihrer Mischungen eingesetzt werden. Ebenso können entsprechende "Oligomere" der Diisocyanate eingesetzt werden (z.B. IPDI-Trimer auf Basis einer Isocyanurat-, Biuret- und/oder Uretdion-Bildung). Des Weiteren ist der Einsatz von Präpolymeren auf Basis der oben genannten Isocyanate möglich.

Es ist auch möglich, Isocyanate einzusetzen, die durch den Einbau von Urethan-, Uretdion-, Isocyanurat, Allophanat- und anderen Gruppen modifiziert wurden, sogenannte modifizierte Isocyanate.

Besonders geeignete organische Polyisocyanate und daher besonders bevorzugt angewendet werden verschiedene Isomere des Toluoldiisocyanat (2,4- und 2,6-Toluoldiisocyanat (TDI), in reiner Form oder als Isomerengemische unterschiedlicher Zusammensetzung), 4,4'-Diphenylmethandiisocyanat (MDI), das so genannte "crude MDI" oder "polymere MDI" (enthält neben dem 4,4`- auch die 2,4`- und 2,2'-lsomeren des MDI und höherkernige Produkte) sowie das als "pure MDI" bezeichnete zweikernige Produkt aus überwiegend 2,4`- und 4,4'-lsomerengemischen bzw. deren Prepolymeren. Beispiele für besonders geeignete Isocyanate sind beispielsweise in EP 1712578, EP 1161474, WO 00/58383, US 2007/0072951, EP 1678232 und der WO 2005/085310 aufgeführt, auf die hier in vollem Umfang Bezug genommen wird.

Optionale Katalysatoren können zusätzlich zum erfindungsgemäßen Katalysator, also dem mindestens einen Zink(II)-Carboxylat, wie zuvor beschrieben, eingesetzt werden. Als möglich Katalysatoren können z.B. auch das mindestens eine tertiäre Amin der Formel (X) sowie z.B. der mindestens eine zusätzliche Trimerisierungskatalysator fungieren, wie zuvor beschrieben.

Geeignete, vorzugsweise geeignete zusätzliche optionale, Katalysatoren im Sinne der vorliegenden Erfindung sind alle Verbindungen, die in der Lage sind die Reaktion von Isocyanaten mit OH-Funktionen, NH-Funktionen oder anderen isocyanat-reaktiven Gruppen sowie mit Isocyanaten selbst zu beschleunigen. Hierbei kann auf die üblichen aus dem Stand der Technik bekannten Katalysatoren zurückgegriffen werden, umfassend z.B. Amine (cyclische, acyclische; Monoamine, Diamine, Oligomere mit einer oder mehreren Aminogruppen), Ammonium-Verbindungen, metallorganische Verbindungen und Metallsalze, vorzugsweise die des Kalium, Zinn, Eisen, Bismuth. Insbesondere können als Katalysatoren Gemische mehrerer Komponenten eingesetzt werden.

Als optionale Schaumstabilisatoren können dazu aus dem Stand der Technik bekannte Stoffe, vorzugsweise Si-freie Surfactants oder auch organomodifizierte Siloxane eingesetzt werden.

Die Verwendung von solchen Substanzen bei der Herstellung von PU- oder PIR-Schaumstoffen ist bekannt. Hierbei können im Rahmen dieser Erfindung alle Verbindungen optional eingesetzt werden, die die Schaumherstellung unterstützen (Stabilisierung, Zellregulierung, Zellöffnung, etc.). Diese Verbindungen sind aus dem Stand der Technik hinreichend bekannt.

Entsprechende, im Sinne dieser Erfindung einsetzbare Siloxane werden z.B. in den folgenden Patentschriften beschrieben: CN 103665385, CN 103657518, CN 103055759, CN 103044687, US 2008/ 0125503, US 2015/0057384, EP 1520870 A1, EP 1211279, EP 0867464, EP 0867465, EP 0275563. Diese vorgenannten Schriften werden hiermit als Referenz eingeführt und gelten als Teil des Offenbarungsgehaltes der vorliegenden Erfindung. Der Einsatz von Polyether-modifizierten-Siloxanen ist besonders bevorzugt.

Die Verwendung von Treibmitteln ist optional, je nachdem welches Verschäumungsverfahren verwendet wird. Es kann mit chemischen und physikalischen Treibmitteln gearbeitet werden. Die Wahl des Treibmittels hängt hier stark von der Art des Systems ab.

In einer besonders bevorzugten Ausführungsform werden keine HFO als Treibmittel eingesetzt.

Je nach Menge des verwendeten Treibmittels kann ein Schaum mit hoher oder niedriger Dichte hergestellt werden. So können Schäume z.B. mit Dichten von 5 kg/m³ bis 900 kg/m³ hergestellt werden. Bevorzugte Dichten sind 8 bis 800 kg/m³, besonders bevorzugt 10 bis 600 kg/m³, insbesondere 30 bis 150 kg/m³.

Als physikalische Treibmittel können entsprechende Verbindung mit passenden Siedepunkten eingesetzt werden. Ebenso können chemische Treibmittel eingesetzt werden, die mit NCO-Gruppen und Freisetzung von Gasen reagieren, wie beispielsweise Wasser oder Ameisensäure. Beispiele für Treibmittel sind verflüssigtes CO2, Stickstoff, Luft, leichtflüchtige Flüssigkeiten, beispielsweise Kohlenwasserstoffe mit 3, 4 oder 5 Kohlenstoff-Atomen, bevorzugt cyclo-, iso- und/oder n-Pentan, Fluorkohlenwasserstoffe, bevorzugt HFC 245fa, HFC 134a und/oder HFC 365mfc, Fluorchlorkohlenwasserstoffe, bevorzugt HCFC 141b, Hydrofluoroolefine (HFO) und/oder Hydrohaloolefine wie z.B. 1234ze, 1234yf, 1233zd(E) oder 1336mzz, Sauerstoff-haltige Verbindungen wie Methylformiat, Aceton und/oder Dimethoxymethan, und/oder Chlorkohlenwasserstoffe, bevorzugt Dichlormethan und/oder 1,2-Dichlorethan.

Geeignete Wasser-Gehalte im Sinne dieser Erfindung hängen davon ab, ob zusätzlich zum Wasser noch ein oder mehrere Treibmittel eingesetzt werden oder nicht. Bei rein Wasser getriebenen Schäumen liegen die Werte vorzugsweise bei 1 bis 20 pphp, werden zusätzlich andere Treibmittel eingesetzt, verringert sich die Einsatzmenge auf vorzugsweise 0,1 bis 5 pphp. Die Abkürzung pphp steht für parts per hundred parts polyol, also für Gewichtsteile pro Hundert Gewichtsteile Polyol. Dies ist eine in der Indrustrie üblicherweise verwendete Methode zur Mengenangabe von Komponenten einer Schaumformulierung.

Als weitere optionale Zusatzstoffe können vorzugsweise alle nach dem Stand der Technik bekannten Substanzen verwendet werden, die bei der Herstellung von Polyurethanen, insbesondere von Polyurethan -oder PIR-Schaumstoffen, Verwendung finden, wie zum Beispiel Vernetzer und Kettenverlängerer, Stabilisatoren gegen oxidativen Abbau (so genannte Antioxidantien), Flammschutzmittel, Tenside, Biozide, zellverfeinernde Additive, Zellöffner, feste Füllstoffe, Antistatik-Additive, Nukleierungsmittel, Verdicker, Farbstoffe, Pigmente, Farbpasten, Duftstoffe, Emulgatoren, usw.

Das erfindungsgemäße Verfahren zur Herstellung von PU- oder PIR-Schaumstoffen, vorzugsweise PU- oder PIR-Hartschaumstoffen, kann nach den bekannten Methoden durchgeführt werden, beispielsweise im Handmischverfahren oder bevorzugt mit Hilfe von Verschäumungsmaschinen. Wird das Verfahren mittels Verschäumungsmaschinen durchgeführt, können Hochdruck- oder Niederdruckmaschinen verwendet werden. Das erfindungsgemäße Verfahren kann sowohl diskontinuierlich als auch kontinuierlich durchgeführt werden.

Eine bevorzugte Polyurethan- oder Polyisocyanurat-Schaumformulierung im Sinne dieser Erfindung ergibt ein Raumgewicht von 5 bis 900 kg/m3 und hat vorzugsweise die in Tabelle 1 genannte Zusammensetzung.

**Tabelle 1:**

| Zusammensetzung einer bevorzugten Polyurethan- oder Polyisocyanurat-Schaumformulierung | |
|---|---|
| Komponente | Gewichtsanteil |
| Polyol | 0,1 bis 100 |
| Amin-Katalysator, umfassend tertiäres Amin der Formel (X) | > 0 bis 5 |
| Optionale zusätzliche Katalysatoren | 0 bis 10 |
| Erfindungsgemäßes Zink(II)-Carboxylat | 0,1 bis 10 |
| Schaumstabilisator (Si-frei oder Si-haltig) | 0 bis 5 |
| Wasser | 0,01 bis 20 |
| Treibmittel | 0 bis 40 |
| Weitere Additive (Flammschutzmittel etc.) | 0 bis 90 |
| Stickstoff-haltige Verbindung V | > 0 bis10 |
| zusätzlicher Trimerisierungskatalysator Isocyanat-Index: 10 bis 1000 | > 0 bis10 |

Für weitere bevorzugte Ausführungsformen und Ausgestaltungen des erfindungsgemäßen Verfahrens sei außerdem auf die zuvor bereits im Zusammenhang mit der erfindungsgemäßen Zusammensetzung gemachten Ausführungen verwiesen.

Wie bereits erwähnt, ist ein weiterer Gegenstand der Erfindung ein PU- oder PIR-Schaumstoff, vorzugsweise PU- oder PIR-Hartschaumstoff, erhältlich durch das genannte Verfahren.

PU- oder PIR-Hartschaumstoff ist ein feststehender technischer Begriff. Der bekannte und prinzipielle Unterschied zwischen Weichschaumstoff und Hartschaumstoff ist, dass ein Weichschaumstoff ein elastisches Verhalten zeigt und damit die Verformung reversibel ist. Der Hartschaumstoff wird demgegenüber dauerhaft verformt. Im Rahmen der vorliegenden Erfindung wird unter PU- oder PIR-Hartschaumstoff insbesondere ein Schaumstoff gemäß DIN 7726:1982-05 verstanden, der eine Druckfestigkeit nach DIN 53 421 / DIN EN ISO 604:2003-12 von vorzugsweise ≥ 20 kPa, bevorzugt ≥ 80 kPa, weiter bevorzugt ≥ 100 kPa, noch weiter bevorzugt ≥ 150 kPa, besonders bevorzugt ≥ 180 kPa aufweist. Weiterhin verfügt der PU- oder PIR-Hartschaumstoff nach DIN EN ISO 4590:2016-12 vorzugsweise über eine Geschlossenzelligkeit von größer 50%, bevorzugt größer 80% und besonders bevorzugt größer 90%. PU- oder PIR-Hartschaumstoff ist im Rahmen der gesamten vorliegenden Erfindung besonders bevorzugt.

Vorzugsweise weist der Polyurethan- oder PIR-Schaumstoff ein Raumgewicht von bevorzugt 5 bis 900 kg/m³, weiter bevorzugt 8 bis 800, besonders bevorzugt 10 bis 600 kg/m³, insbesondere 30 bis 150 kg/m³ auf.

Es können vorzugsweise überwiegend geschlossenzellige Schaumstoffe hergestellt werden. Die Geschlossenzelligkeit beträgt vorzugsweise > 80%, bevorzugt > 90 %.

Die erfindungsgemäßen PU- oder PIR-Schaumstoffe können bevorzugt als oder zur Herstellung von Isoliermaterialien, vorzugsweise Dämmplatten, Kühlschränken, Isolierschäumen, Dachhimmeln, Verpackungsschäumen oder Sprühschäumen verwendet werden.

Insbesondere in der Kühlhaus-, Kühlgeräte- und Hausgeräteindustrie; z. B. zur Herstellung von Dämmplatten für Dächer und Wände, als Isoliermaterial in Containern und Lagerhäusern für tiefgekühlte Ware sowie für Kühl- und Gefriergeräte, können die erfindungsgemäßen PU- oder PIR-Schaumstoffe mit Vorteil eingesetzt werden.

Weitere bevorzugte Anwendungsfelder liegen im Fahrzeugbau, insbesondere zur Herstellung von Fahrzeughimmel, Karosserieteilen, Innenverkleidungen, Kühlfahrzeugen, Großcontainern, Transportpaletten, Verpackungslaminaten, in der Möbelindustrie, z.B. für Möbelteile, Türen, Verkleidungen, in Elektronikanwendungen.

Vorzugsweise können erfindungsgemäße PU- oder PIR-Schaumstoffe (Polyurethan- oder Polyisocyanuratschaumstoffe) als Isoliermaterial für Kühlapparaturen verwendet werden.

Ein weiterer Gegenstand der Erfindung liegt in der Verwendung des PU- oder PIR-Schaumstoffs als Isolationsmaterial in der Kältetechnik, in Kühlmöbeln, im Bau-, Automobil-, Schiffbau- und/oder Elektronikbereich, als Dämmplatten, als Sprühschaum, als Einkomponentenschaum.

Die Erfindung wird nachfolgend anhand von Beispielen weiter beispielhaft beschrieben, ohne die Erfindung dadurch in irgendeiner Form zu beschränken. Sind Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden Mittelwerte angegeben, so handelt es sich, wenn nicht anderes angegeben, um Gewichtsmittel. Werden Parameter angegeben, die durch Messung bestimmt wurden, so wurden die Messungen, wenn nicht anders angegeben, bei einer Temperatur von 25 °C und Normaldruck (vorzugsweise 101325 Pa) durchgeführt.

### BEISPIELE:

Es wurden folgende Rohstoffe eingesetzt:
   Stepanpol^{®} PS 2352: Polyesterpolyol der Firma Stepan
   Daltolac^{®} R 471: Polyetherpolyol der Firma Huntsman
   TCPP: Tris(2-chlorisopropyl)phosphat der Firma ICL
   KOSMOS^{®} 75 von Fa. Evonik Operations GmbH, Katalysator auf Basis Kaliumoctoat
   KOSMOS^{®} 33 MEG von Fa. Evonik Operations GmbH, Katalysator auf Basis Kaliumacetat
   POLYCAT^{®} 5 von Fa. Evonik Operations GmbH, Amin-Katalysator
   POLYCAT^{®} DP von Fa. Evonik Operations GmbH, Amin-Katalysator
   POLYCAT^{®} 9 von Fa. Evonik Operations GmbH, Amin-Katalysator
   POLYCAT^{®} 206 von Fa. Evonik Operations GmbH, Amin-Katalysator
   POLYCAT^{®} 77 von Fa. Evonik Operations GmbH, Amin-Katalysator
   TEGOAMIN^{®} BDE von Fa. Evonik Operations GmbH, Amin-Katalysator
   DABCO^{®} T von Fa. Evonik Operations GmbH, Amin-Katalysator
   DABCO^{®} NE 300 von Fa. Evonik Operations GmbH, Amin-Katalysator
   Amin Nr. 1: bestehend aus N,N'-diisopropyl-N,N'-dimethyl-bis (aminoethyl) ether, N,N,N`-triisopropyl-N'-methyl-bis (aminoethyl) ether wie in WO 2023/043980 A2 beschrieben in Beispiel 4 als Stuktur V und VI
   Amin Nr. 2: bestehend aus N,N'-diisobutyl-N,N'-dimethyl-bis (aminoethyl) ether wie in WO 2023/043980 A2 beschrieben in Beispiel 8 als Stuktur XVII
   MDI (44V20): Desmodur^{®} 44V20L der Fa. Covestro, Diphenylmethan-4,4'-diisocyanat (MDI) mit Isomeren und höherfunktionellen Homologen
   Tegostab^{®} B 8460 von Fa. Evonik Operations GmbH, schaumstabilisierendes Surfactant.

### Herstellung von Zink-haltigen Zubereitungen:

Es werden verschiedene Zubereitungen hergestellt, die dann in den Verschäumungen zu erfindungsgemäßen (oder nicht erfindungsgemäßen) Zusammensetzungen kombiniert werden können.

Die entsprechenden Komponenten können vorformuliert oder als einzelne Komponenten der zu verschäumenden Reaktionsmischung zugegeben werden.

Erfindungsgemäß sind die Beispiele, die Zink enthalten.

Rohstoffe zur Herstellung der Zink-haltigen Zubereitungen:
Zn-Ac: Zinkacetat Dihydrat (erhältlich bei Sigma-Aldrich)
Zn-Riz: Zink-Rizinoleat, (erhältlich Evonik als TEGODEO^{®} PY 88 G)
EDA-PO: N,N,N',N'-tetrakis(2-hydroxypropyl)ethylendiamin - (beschrieben in WO 2022/218657 A1 und erhältlich bei BASF)
V1: 2,4,6-tris[(dimethylamino)methyl]phenol (erhältlich bei Sigma-Aldrich)
DEG: Diethylenglykol.

### Herstellung der Zubereitungen:

Es wurden die flüssigen Komponenten wie DEG, EDA-PO oder V1 vorgelegt und anschließend die Zinksalze Zn-Ac oder Zn-Riz zugegeben und bei ca. 50°C gerührt, um eine klare Mischung zu erhalten.

In Tabelle 1 sind die Zusammensetzungen der Zubereitungen zusammengefasst mit den Gewichtsteilen der einzelnen Komponenten.

Es wurden die Viskositäten mit einem Brookfieldviskosimeter bei Raumtemperatur 23°C bestimmt.

**Tabelle 1**

| Übersicht über Zink-haltige Zubereitungen (ZZ) A bis L und deren Viskositäten | | | | | | | | |
|---|---|---|---|---|---|---|---|---|
| Name | | Zinksalz | Gew.-Teile | | Gew.-Teile | | Gew. -Teile | Viskosität |
| A | erf. | Zn-Ac | 40 | V1 | 60 | | | 200 Pa·s |
| B | n. erf. | Zn-Ac | 40 | EDA-PO | 60 | | | >500 Pa·s |
| C | erf. | Zn-Riz | 70 | V1 | 30 | | | 60 Pa·s |
| D | n. erf. | Zn-Riz | 70 | EDA-PO | 30 | | | >200 Pa·s |
| E | erf. | Zn-Riz | 56 | V1 | 12 | DEG | 32 | 7260 mPa·s |
| F | erf. | Zn-Ac | 30 | V1 | 40 | DEG | 30 | 2100 mPa·s |
| G | n. erf. | Zn-Ac | 30 | EDA-PO | 40 | DEG | 30 | 8800 mPa·s |
| H | erf. | Zn-Ac | 40 | V1 | 30 | DEG | 30 | 3060 mPa·s |
| J | erf. | Zn-Ac | 45 | V1 | 30 | DEG | 25 | 7020 mPa·s |
| K | erf. | Zn-Ac | 30 | V1 | 20 | DEG | 50 | 390 mPa·s |
| L | n. erf. | Zn-Ac | 30 | EDA-PO | 20 | DEG | 50 | 370 mPa·s |

Hierbei sind die Zubereitungen B, D,G und L nicht erfindungsgemäß.

(erf. = erfindungsgemäß; n. erf. = nicht erfindungsgemäß)

Mit V1 ließen sich Gemische mit höheren Zink-Gehalten herstellen, die nicht auskristallisierten und niedrigere Viskositäten hatten als es mit EDA-PO möglich war.

So waren bei 40% Zink-Acetat die Viskositäten mit EDA-PO größer als 500 Pa·s (Zusammensetzung B) und mit V1 bei ca. 200 Pa·s (Zubereitung A).

Bei Verwendung von Zink-Rizinoleat zeigte sich der gleiche Trend mit größer 200 Pa·s (Zusammensetzung D) und 60 Pa·s (Zubereitung C).

Solch hohe Viskositäten sind in der Praxis kaum verarbeitbar, aber man erkennt den Vorteil von V1, welches zu niedrigeren Viskositäten beiträgt.

Für eine gute Pumpbarkeit sind Viskositäten von kleiner 10000 mPa·s oder besser von kleiner 3000 mPa·s sinnvoll.

Mit Zubereitungen wie F und G erkennt man den Vorteil von V1 gegenüber EDA-PO ebenso. Ein flüssiges Produkt mit einer Viskosität von 2100 mPa·s ist deutlich einfacher zu verarbeiten als ein Material mit 8800 mPa·s.

Mit V1 als Abmischkomponente war es möglich Zink-Acetat Dihydrat in Anteilen von 45% in einer Mischung zu haben und eine Viskosität von 7000 mPa·s, wie in Zusammensetzung J ersichtlich; also mit niedrigerer Viskosität als eine Mischung wie G mit 30% Zink-Acetat Dihydrat.

Zubereitung L zeigte außerdem trotz recht niedriger Viskosität von kleiner 1000 mPa·s nach einigen Tagen Ausfällungen. Dagegen war die Zubereitung K, die V1 enthält anstatt EPA-PO über Wochen stabil und klar löslich.

Hieraus ist klar ersichtlich, dass die erfindungsgemäßen Zubereitungen Vorteile haben gegenüber den bisher bekannten Zusammensetzungen.

**Zusätzliche Trimerisierungskatalysatoren, die Teil einer Zusammensetzung sein können:**
Komponente M: Kalium Acetat basierender Trimerisierungskatalysator: Kosmos^{®} 33 MEG von Evonik Operations GmbH.
Komponente N: Kalium Octoat basierender Trimerisierungskatalysator: Kosmos^{®} 75 von Evonik Operations GmbH.
Komponente O: Kalium Neocanoat basierender Trimerisierungskatalysator: Kosmos^{®} K 65 LO von Evonik Operations GmbH
Komponente P: Kalium-Propionat basierender Trimerisierungskatalysator: KPROP 14 von Schill&Seilacher

### Herstellung von PU- und PIR- Schaumstoffen:

Die Durchführung der Verschäumungen erfolgte im Handmischverfahren. Dazu wurden die erfindungsgemäßen Verbindungen, Polyole, Flammschutzmittel, erfindungsgemäße oder nicht erfindungsgemäße Katalysatoren, Wasser, Siloxan-Surfactant und Treibmittel in einen Becher eingewogen und mit einem Tellerrührer (6cm Durchmesser) 30 s bei 1000 Upm vermischt. Durch erneutes Abwiegen wurde die beim Mischvorgang verdunstete Treibmittelmenge bestimmt und wieder ergänzt. Anschließend wurde das Isocyanat (MDI) zugegeben, die Reaktionsmischung mit dem beschriebenen Rührer 5 s bei 3000 Upm verrührt.

Die Reaktionsmischungen wurden in entsprechende Becher mit einem Durchmesser an der Oberkannte von 20 cm eingetragen um frei gestiegene Schäume zu erhalten. Die Menge der Reaktionsmischung wurde so gewählt, dass die Spitze der Schaumkuppe am Ende 10 bis 15 cm über der Oberkante des Bechers lag.

Während des Aufschäumens wurde die Gelzeit bestimmt, um den Einfluss der Katalysatoren auf die Geschwindigkeit des Schäumens zu beurteilen.

Nach 2 Minuten wurden die Schaumkuppen an der Becheroberkante abgeschnitten, so dass man eine runde Schaumfläche erhielt. An dieser Oberfläche wurden die Eindrückhärten der Schäume bestimmt.

### Bestimmungsmethode der Eindrückhärte:

Hierzu wurde die Kraft gemessen, um einen Stempel mit 4 cm Durchmesser in den Schaum einzudrücken. Es wurden die Eindrückkräfte bei 5 mm Eindrücktiefe gemessen. Die Messung erfolgte im Abstand von 90 Sekunden also nach 3, 5 ½, 8 und 10 ½ Minuten, wobei der Stempel an 4 verschiedenen Punkten der Schnittfläche, in kreisförmiger Anordnung, eingedrückt wurde.

### Bestimmungsmethode der Stauchhärte:

Die Stauchhärten der Schaumstoffe werden an würfelförmigen Probekörpern mit 5 cm Kantenlänge nach DIN EN ISO 844:2014-11 bis zu einer Stauchung von 10% gemessen (angegeben wird die in diesem Messbereich maximal aufgetretene Druckspannung).

In Tabelle 2 sind die verwendeten Schaumformulierungen (Form.1 bis Form. 9) zusammengefasst. Es sind die jeweiligen Gewichtsteile der Bestandteile angegeben.

Diese Formulierung wurden dann noch mit weiteren Katalysatoren versetzt, was dann entsprechend erfinderischen oder nicht erfinderischen Zusammensetzungen entspricht. Diese weiteren Katalysatoren und die Ergebnisse dazu sind dann in Tabelle 3 zusammengefasst.

**Tabelle 2:**

| Schaum Formulierungen (PIR und PUR) | | | | | |
|---|---|---|---|---|---|
| Formulierung | Form. 1 | Form. 2 | Form. 3 | Form. 4 | Form. 5 |
| Daltolac^{®} R 471 | | | | | |
| PS 2352 | 100 | 100 | 100 | 100 | 100 |
| POLYCAT^{®} 5 | 0,5 | | | | |
| POLYCAT^{®} 9 | | 0,55 | | | |
| POLYCAT^{®} 206 | | | 0,9 | | |
| POLYCAT^{®} 77 | | | | 0,65 | |
| TEGOAMIN^{®} BDE | | | | | 0,8 |

| Weitere Kats | siehe Tabelle 3 | siehe Tabelle 3 | siehe Tabelle 3 | siehe Tabelle 3 | siehe Tabelle 3 |
|---|---|---|---|---|---|
| TEGOSTAB^{®} B 8460 | 2 | 2 | 2 | 2 | 2 |
| TCPP | 15 | 15 | 15 | 15 | 15 |
| Wasser | 0,5 | 0,5 | 0,5 | 0,5 | 0,5 |
| n-Pentan | 14 | 14 | 14 | 14 | 14 |
| Cyclo-Pentan | | | | | |
| MDI (44V20) | ca. 190 | ca. 190 | ca. 190 | ca. 190 | ca. 190 |
| Index | 255 | 255 | 255 | 255 | 255 |

**Tabelle 2**

| (Fortsetzung) Formulierungen | | | | | | |
|---|---|---|---|---|---|---|
| Formulierung | Form. 6 | Form. 7 | Form. 8 | Form. 9 | Form. 10 | Form. 11 |
| Daltolac^{®} R 471 | | | | | 100 | 100 |
| PS 2352 | 100 | 100 | 100 | 100 | | |
| POLYCAT^{®} 5 | | | | | 2,1 | |
| POLYCAT^{®} DP | | | | | | 3,5 |
| DABCO^{®} T | 0,5 | | | | | |
| POLYCAT^{®} NE 300 | | 1,1 | | | | |
| Amin Nr. 1 | | | 1 | | | |
| Amin Nr. 2 | | | | 1,1 | | |

| Weitere Kats | siehe Tabelle 3 | siehe Tabelle 3 | siehe Tabelle 3 | siehe Tabelle 3 | siehe Tabelle 3 | siehe Tabelle 3 |
|---|---|---|---|---|---|---|
| TEGOSTAB^{®} B 8460 | 2 | 2 | 2 | 2 | 1,4 | 1,4 |
| TCPP | 15 | 15 | 15 | 15 | | |
| Wasser | 0,5 | 0,5 | 0,5 | 0,5 | 2,5 | 2,5 |
| n-Pentan | 14 | 14 | 14 | 14 | | |
| Cyclo-Pentan | | | | | 13 | 13 |
| MDI (44V20) | ca. 190 | ca. 190 | Ca. 190 | Ca. 190 | Ca. 190 | Ca. 190 |
| Index | 255 | 255 | 255 | 255 | 130 | 130 |

### Verschäumungsergebnisse:

Tabelle 3:
Zusammenfassung der Verschäumungsversuche mit verschiedenen Katalysatoren und Schaumformulierungen.

Angegeben sind die verwendeten Komponenten (Kmp. A-P, je nach Zusammensetzung erfinderisch oder nicht), deren Dosierung (Gewichtsteile) (bezeichnet als Dos.), die verwendete Formulierung aus Tabelle 2, die Gelzeit (GZ) in Sekunden, sowie die Eindrückhärten in Newton nach der angegebenen Zeit in Minuten (nach dem Mischen mit MDI).

Die Katalysator-Zusammensetzungen ZZ, die entweder kein V1 oder kein Zink enthalten, sind nicht erfinderisch.

Die Komponenten an Trimerisierungskatalysatoren, Amine und ZZ können auch vorgemischt werden. Hier in den Beispielen werden der Übersicht halber die Komponenten wie z.B. tert. Amine, Zink-Salze, Stickstoff-haltige Verbindung V1), Trimerisierungskatalysatoren über die Formulierungen in Tabelle 2 und die zusätzlichen Komponenten in Tabelle 3 getrennt dargestellt.

**Tabelle 3:**

| | | | | | | | Kräfte in N nach Zeit von | | | |
|---|---|---|---|---|---|---|---|---|---|---|
| Bsp. | Kmp. | Dos. | Kmp. | Dos. | Form. Nr. | GZ /sec. | 3 min | 5'30‴ | 8 min | 10`30" min |
| 1 | K | 1 | M | 1,4 | 1 | 61 | 121 | 260 | 367 | 424 |
| Vgl.1 | | | M | 1,1 | 1 | 64 | 85 | 194 | 284 | 375 |
| Vgl.2 | L | 1 | M | 2 | 1 | 59 | 112 | 260 | 365 | 430 |
| 2 | K | 2 | M | 2 | 1 | 59 | 219 | 328 | 413 | 479 |
| 3 | E | 1 | M | 1,1 | 1 | 61 | 96 | 258 | 329 | 394 |
| 4 | A | 1 | M | 0,9 | 1 | 61 | 72 | 192 | 286 | 373 |
| 5 | C | 1 | M | 1,1 | 1 | 60 | 75 | 226 | 311 | 385 |
| 6 | J | 0,5 | M | 1.3 | 1 | 65 | 87 | 258 | 334 | 421 |
| 7 | J | 0,3 | M | 1,2 | 1 | 62 | 102 | 248 | 335 | 407 |
| 8 | J | 0,25 | P | 1,0 | 1 | 63 | 86 | 234 | 415 | 454 |
| 9 | J | 0,425 | O | 1,7 | 1 | 61 | 109 | 256 | 346 | 396 |
| Vgl.3 | | | M | 1,1 | 2 | 60 | 75 | 183 | 256 | 355 |
| 10 | K | 1 | M | 1,4 | 2 | 61 | 114 | 236 | 354 | 412 |
| 11 | J | 0,5 | M | 1,2 | 2 | 60 | 118 | 232 | 356 | 424 |
| Vgl.4 | | | M | 1,1 | 3 | 61 | 83 | 190 | 267 | 371 |
| 12 | K | 1 | M | 1,4 | 3 | 61 | 123 | 253 | 347 | 413 |
| 13 | J | 0,5 | M | 1,2 | 3 | 60 | 119 | 261 | 364 | 418 |
| Vgl.5 | | | M | 1,1 | 4 | 59 | 93 | 189 | 284 | 362 |
| 14 | K | 1 | M | 1,4 | 4 | 61 | 132 | 235 | 376 | 435 |
| 15 | J | 0,5 | M | 1,2 | 4 | 59 | 127 | 263 | 356 | 419 |
| Vgl.6 | | | M | 1,1 | 5 | 60 | 87 | 198 | 273 | 367 |
| 16 | K | 1 | M | 1,4 | 5 | 60 | 117 | 259 | 361 | 421 |
| 17 | J | 0,5 | M | 1,2 | 5 | 60 | 116 | 264 | 365 | 419 |
| Vgl.7 | | | M | 1,1 | 6 | 61 | 85 | 187 | 279 | 363 |
| 18 | K | 1 | M | 1,4 | 6 | 61 | 120 | 269 | 371 | 420 |
| 19 | J | 0,5 | M | 1,2 | 6 | 59 | 124 | 270 | 359 | 409 |
| Vgl.8 | | | M | 1,1 | 7 | 60 | 87 | 191 | 275 | 369 |
| 20 | K | 1 | M | 1,4 | 7 | 60 | 130 | 258 | 373 | 424 |
| 21 | J | 0,5 | M | 1,2 | 7 | 61 | 126 | 266 | 365 | 418 |
| Vgl.9 | | | M | 1,1 | 8 | 60 | 93 | 199 | 283 | 371 |
| 22 | K | 1 | M | 1,4 | 8 | 59 | 125 | 265 | 371 | 422 |
| 23 | J | 0,5 | M | 1,2 | 8 | 60 | 131 | 259 | 378 | 433 |
| Vgl.10 | | | M | 1,1 | 9 | 60 | 88 | 194 | 277 | 368 |
| 24 | K | 1 | M | 1,4 | 9 | 61 | 134 | 267 | 369 | 429 |
| 25 | J | 0,5 | M | 1,2 | 9 | 61 | 122 | 271 | 372 | 428 |
| Vgl. 11 | | | | | 10 | 56 | 72 | 151 | 234 | 309 |
| 26 | K | 1 | | | 10 | 56 | 172 | 281 | 348 | 411 |
| Vgl 12 | | | | | 11 | 52 | 107 | 183 | 255 | 366 |
| 27 | K | 1 | | | 11 | 44 | 244 | 362 | 420 | 445 |

In Beispiel 1 wurden deutlich höhere Eindrückhärten erzielt als in Vgl. 1, wo keine erfindungsgemäße Zink-haltige Zusammensetzung verwendet wurde.

Auch in Vgl.2 zeigt sich der Vorteil der erfindungsgemäßen Zusammensetzung. Es müssen in Kombination mit L (nicht erfinderisch) 2 Teile M eingesetzt werden, um die gleiche Gelzeit und Durchhärtung zur erhalten im Vergleich zu Beispiel 1. Hier kann mit 1,4 Teilen M eine Gelzeit von 60 Sekunden erreicht werden und vergleichbare Durchhärtungen/Eindrückkräfte.

Daher ist K effizienter als Katalysator als L.

Bei Beispiel 7 wurden nur 0,3 Teile von J und 1,2 Teile Kosmos 33 eingesetzt um eine Gelzeit von 60 Sekunden und die verbesserten Eindrückhärten zu erreichen.

Daraus ist der Vorteil ersichtlich, dass mit den Zink-Phenol-Kombinationen effizientere Katalysatoren zur Durchhärtung bereitgestellt werden können.

Auch mit P und O erreichte man mit geringen Dosierungen von J die gewünschten Effekte auf die Schaumhärte wie aus den Beispielen 8 und 9 zu erkennen ist.

Die weiteren Versuche in verschiedenen Formulierungen zeigen den gleichen Effekt.

Hieraus ist ersichtlich, dass die Erfindung in diversen Formulierungen eine verbesserte Aushärtung des Schaumes ermöglicht. Dabei können die Gelzeiten teilweise sogar verlängert werden oder mit gleichbleibenden Gelzeiten die positiven Effekte auf die Durchhärtung weiter verbessert werden. Dies ist ein enormer Vorteil, da durch den geringen Einfluss auf die Gelzeit die Verarbeitbarkeit der Reaktionsmischung erhalten bleibt, z.B. hinsichtlich der Fließfähigkeit der schäumenden Mischung, und gleichzeitig die Aushärtung des Schaumes beschleunigt wird.

Aus den Versuchen ist klar ersichtlich, dass die erfindungsgemäßen Zink-haltigen Zubereitungen bzw. erfindungsgemäßen Zusammensetzungen zu einer verbesserten Aushärtung des Schaums führen. Die zuvor beschriebenen sehr guten Resultate für die Eindrückhärten der erfindungsgemäßen Schäume korrespondieren mit denen der Stauchhärte.

## Patentansprüche

1. Zusammensetzung zur Herstellung von Polyurethan- oder Polyisocyanurat-Schaumstoff, umfassend
a) eine Polyisocyanat-Komponente,
b) eine Polyolkomponente,
c) mindestens ein Zink(II)-Carboxylat, mit der Maßgabe, dass das enthaltene Zink(II)-Carboxylat in stöchiometrischer Form eingesetzt wird, also mit Zn(II) und Carboxylat im molaren Verhältnis von 1 zu 2,
d) mindestens ein tertiäres Amin der Formel (X) 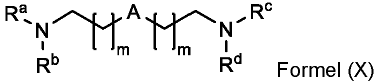 mit
m jeweils unabhängig voneinander 1 oder 2,
A ist O, S oder N-R^{e},
R^{a}, R^{b}, R^{c}, R^{d} und R^{e}, jeweils unabhängig voneinander gleiche oder verschiedene lineare, verzweigte oder cyclische Alkylreste mit 1 bis 20 Kohlenstoffen,
e) optional mindestens einen Schaumstabilisator,
f) optional mindestens ein Treibmittel,
**dadurch gekennzeichnet, dass** die Zusammensetzung zusätzlich
g) mindestens eine Stickstoff-haltige Verbindung V enthält, welche mindestens zwei N-Atome aufweist und bei der es sich um ein modifiziertes Phenol handelt.

2. Zusammensetzung nach Anspruch 1, **dadurch gekennzeichnet, dass** das mindestens eine Zink(II)-Carboxylat ausgewählt ist aus der Gruppe bestehend aus Zink(II)-acetat, Zink(II)-propionat, Zink(II)-pivalat, Zink(II)-2-ethylhexanoat (Zink(II)-octoat), Zink(II)-isononanoat (Zink(II)-3,5,5-trimethylhexanoat), Zink(II)-neodecanoat, Zink(II)-ricinoleat, Zink(II)-palmitat, Zink(II)-stearat, Zink(II)-oleat, Zink(II)-laurat, Zink(II)-napthenat, Zink(II)-benzoat, Zink(II)-laktat, Zink(II)-glycinat, Zink(II)-hippurat, Zink(II)-citrat, und Zink(II)-Seifen, wobei der Einsatz von Zink(II)-acetat und/oder Zink(II)-ricinoleat ganz besonders bevorzugt ist.

3. Zusammensetzung nach einem der Ansprüche 1 oder 2, **dadurch gekennzeichnet, dass** die mindestens eine Stickstoff-haltige Verbindung V ausgewählt ist aus der Gruppe bestehend aus 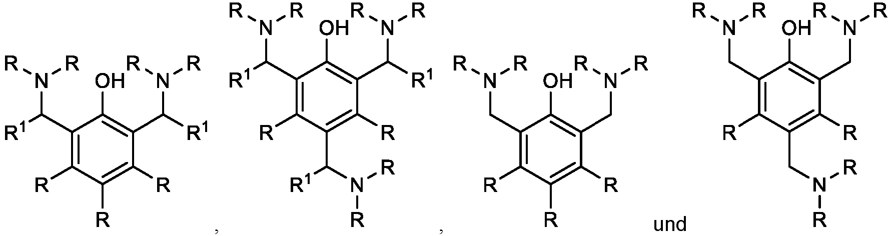 wobei
R = jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann, optional enthaltend Heteroatome wie O oder N,
R¹ = jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann.

4. Zusammensetzung nach einem der Ansprüche 1 bis 3, **dadurch gekennzeichnet, dass** die mindestens eine Stickstoff-haltige Verbindung V durch Umsetzung von Phenol mit Aldehyd, vorzugsweise Aldehyd mit 1 bis 20 Kohlenstoffen, bevorzugt Formaldehyd, Acetaldehyd, Propionaldehyd, Butyraldehyde, Benzaldehyd, Zimtaldehyd, Trimethylhexanal, Pelargonaldehyd, Acrolein und/oder Furfural und Amin im Sinne der Mannich-Reaktion hergestellt wird,
wobei als Ausgangsmaterial auch modifizierte Phenole verwendet werden können, die mindestens einen Substituenten am aromatischen Kern tragen, wie vorzugsweise Kresol, Xylenole, Propylphenole, Styrylphenole, Alkylphenole und/oder Cardanol, ebenso können vorzugsweise auch Resorcin und/oder Catechol-basierende Strukturen als modifizierte Phenole eingesetzt werden,
bevorzugt wird die mindestens eine Stickstoff-haltige Verbindung V durch Umsetzung von Phenol mit Formaldehyd und mindestens einem primären oder sekundären Amin, vorzugsweise ausgewählt aus der Gruppe bestehend aus Dimethylamin, Diethylamin, Dipropylamin, Diisopropylamin, Dibutylamin, Ethanolamin, Diethanolamin, Trimethylethandiamin, Isophorondiamin, Dimethylpropylamin, Diethylentriamin, Triethylentetramin, Methylhydroxyethylamin, Dimethylaminopropylamin, Pyrrolidin, Pyrrol, Morpholin und/oder Piperazin, N,N-Dimethyl-2-(2-methylaminoethoxy)ethan-1-amin, N-[2-[2-(Dimethylamino)ethoxy]ethyl]-N-methyl-1,3-propanediamine, Diisopropyldimethyldiethylentriamin, Bis-(dimethylaminopropyl)amine Trimethylaminoethylethanolamin, N,N'-Diisopropyl-N-methyl-bis (aminoethyl) ether und N,N'-diisobutyl-N-methyl-bis (aminoethyl) ether hergestellt, wobei als Ausgangsmaterial wiederum auch modifizierte Phenole verwendet werden können.

5. Zusammensetzung nach einem der Ansprüche 1 bis 4, **dadurch gekennzeichnet, dass** die mindestens eine Stickstoff-haltige Verbindung V ausgewählt ist aus der Gruppe bestehend aus 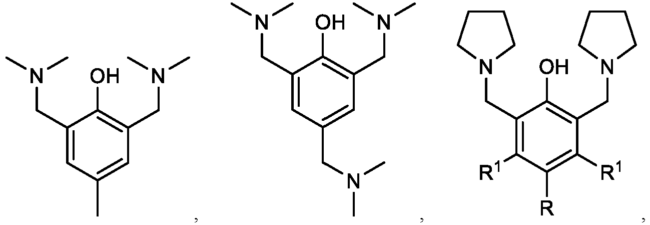 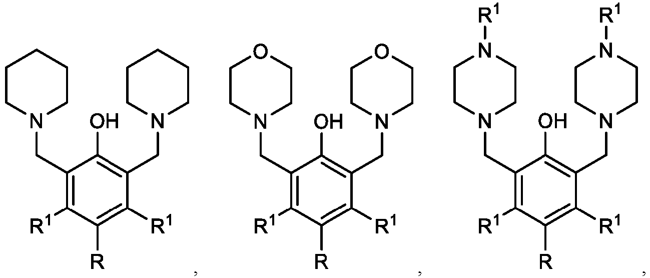 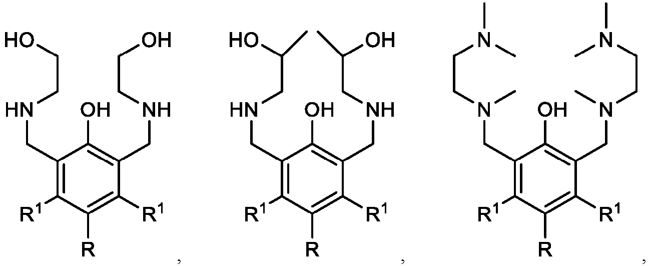 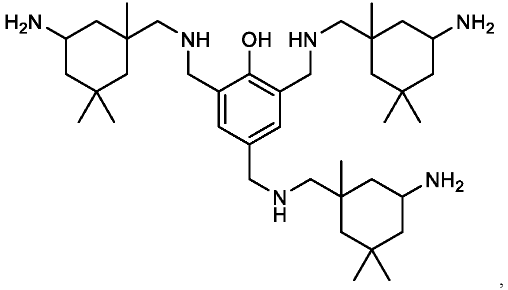 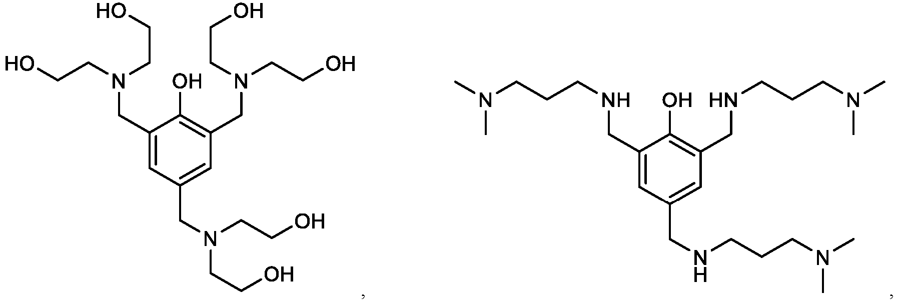 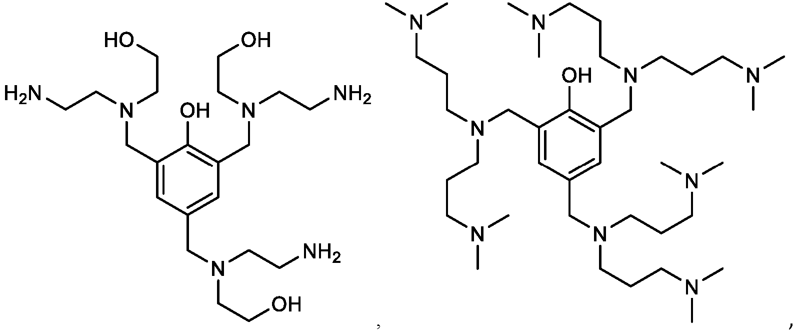 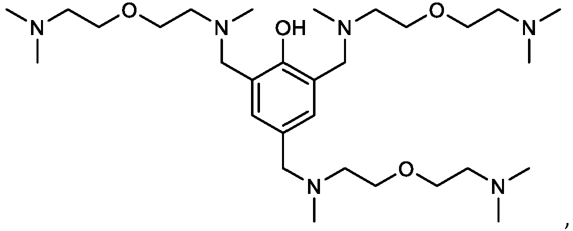 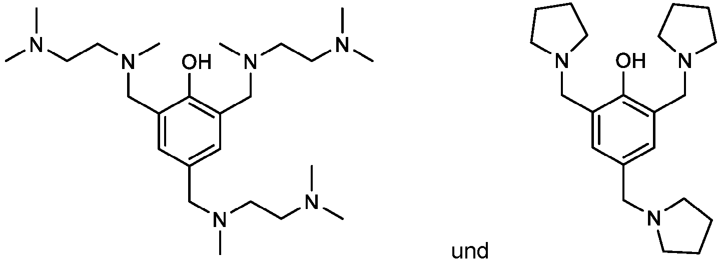 wobei
R = jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann, optional enthaltend Heteroatome wie O oder N,
R¹ = jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann,
besonders bevorzugt ist der Einsatz von 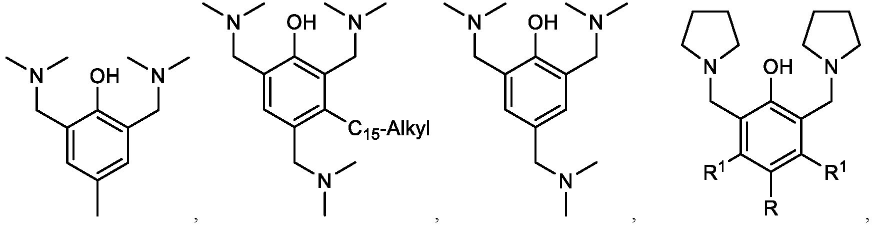 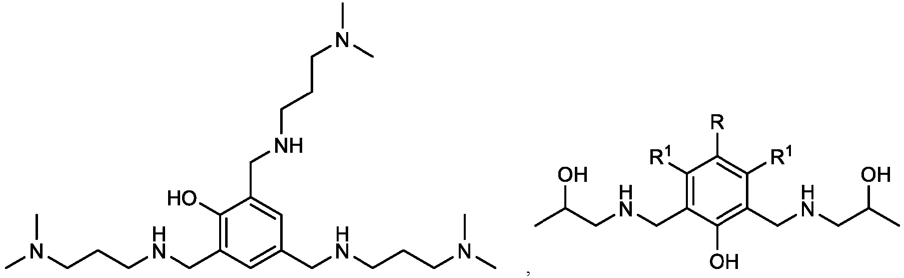 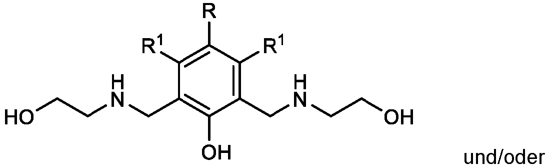 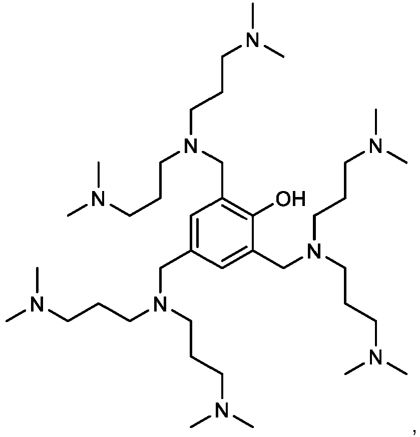
mit
R = jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann, optional enthaltend Heteroatome wie O oder N,
R¹ = jeweils unabhängig voneinander H oder linearer, verzweigter oder cyclischer Kohlenwasserstoffrest mit 1 bis 20 Kohlenstoffatomen, der gesättigt, ungesättigt oder aromatisch sein kann,
ganz besonders bevorzugt ist der Einsatz von 2,6-bis[(dimethylamino)methyl]-4-methyl-phenol, 2,4,6-tris[(dimethylamino)methyl]cardanol, 2,4,6-tris[(dimethylamino)methyl]phenol, 2,6-bis[(dimethylamino)methyl]-cardanol, 2,4,6-tris-[(hydroxyethylamino)methyl]phenol, 2,4,6-tris[(hydroxypropylamino)methyl]phenol und/oder 2,4,6-tris[(dimethylaminopropylamino)methyl]phenol.

6. Zusammensetzung nach einem der Ansprüche 1 bis 5 **dadurch gekennzeichnet, dass** insgesamt enthaltenes Zink(II)-Carboxylat und insgesamt enthaltene Sticktoff-haltige Verbindung V im Mengenverhältnis von 1 zu 0,5 bis 1 zu 5 Gewichtsteilen zueinander vorliegen.

7. Zusammensetzung nach einem der Ansprüche 1 bis 6, **dadurch gekennzeichnet, dass** das mindestens eine tertiäre Amin der Formel (X) ausgewählt ist aus der Gruppe bestehend aus Pentamethyldiethylentriamin, Bis-(2-dimethylaminoethyl)ether, Tris(dimethylaminopropyl)-amin, N-[2-[2-(Dimethylamino)ethoxy]ethyl]-N-methyl-1,3-propanediamin, Diisopropyltrimethyldiethylentriamin, Bis-(dimethylaminopropyl)methylamin, Trimethylaminoethylethanolamin, Bis-(2-isopropylmethylaminoethyl)ether, Bis-(2-isobutylmethylaminoethyl)ether, N,N'-Diisopropyl-N,N'-dimethyl-bis(aminoethyl)ether, N,N,N'-Triisopropyl-N'-methyl-bis(aminoethyl)ether und N,N'-Diisobutyl-N,N'-dimethyl-bis(aminoethyl)ether.

8. Zusammensetzung nach einem der Ansprüche 1 bis 7, **dadurch gekennzeichnet, dass** zudem noch mindestens ein zusätzlicher Trimerisierungskatalysator enthalten ist, vorzugsweise ausgewählt aus Carboxylaten von Ammonium, Kalium und/oder anderen Alkali- oder Erdalkalimetallen,
bevorzugt ausgewählt aus Kalium-Carboxylaten und Carboxylaten von Ammonium-Kationen, ganz besonders bevorzugt ausgewählt aus der Gruppe bestehend aus Kaliumacetat, Kaliumformiat, Kaliumpropionat, Kaliumbutanoat, Kaliumpentanoat, Kaliumhexanoat, Kaliumheptanoat, Kalium-2-ethylhexanoat, Kaliumpivalat, Kaliumoctoat, Kaliumbutyrat, Kalium isobutyrat, Kalium nonanoat, Kaliumdecanoat, Kaliumrizinoleat, Kaliumstearat, Kaliumneodecanoat und Carboxylaten von Tetramethylammonium, Tetraethylammonium, Triethylmethylammonium, Tetrapropylammonium, Tetrabutylammonium, Dimethyldiallylammonium, Trimethyl-(2-hydroxypropyl)ammonium, Triethyl-(2-hydroxypropyl)ammonium, Tripropyl-(2-hydroxypropyl)ammonium, Tributyl-(2-hydroxypropyl)ammonium, Trimethyl-(2-hydroxyethyl)ammonium, Triethyl-(2-hydroxyethyl)ammonium, Tripropyl-(2-hydroxyethyl)-ammonium, Tributyl-(2-hydroxyethyl)ammonium, Dimethylbenzyl-(2-hydroxyethyl)ammonium und/oder Dimethylbenzyl-(2-hydroxypropyl)ammonium, wobei die Carboxylate vorzugweise Acetate, Propionate, Butanoate, Pentanoate Pivalate, Octoate, Nonanoate, Decanoate, Neodecanoate, Rizinoleate und/oder Stearate sind.

9. Zusammensetzung nach einem der Ansprüche 1 bis 8, **dadurch gekennzeichnet, dass** die gesamt enthaltene Polyolkomponente zu 70 bis 100 Gew.-% aus aromatischen Polyesterpolyolen besteht, mit OH-Zahlen von 150 bis 400 mg KOH/g, bevorzugt 170 bis 350 KOH/g, ganz besonders bevorzugt 180 bis 300 mg KOH/g.

10. Zusammensetzung nach einem der Ansprüche 1 bis 9, **dadurch gekennzeichnet, dass** sie mindestens ein physikalisches Treibmittel umfasst, vorzugsweise ausgewählt aus der Gruppe der Kohlenwasserstoffe mit 3, 4 und/oder 5 Kohlenstoff-Atomen, bevorzugt ausgewählt aus der Gruppe bestehend aus n-Butan, cyclo-, iso- und n-Pentan.

11. Zink(II)-Carboxylat-haltige Zubereitung, geeignet zur Katalyse bei der Herstellung von PU- oder PIR-Schaumstoff, **dadurch gekennzeichnet, dass** sie umfasst:
i) mindestens ein Zink(II)-Carboxylat, vorzugsweise wie in Anspruch 1 oder 2 definiert, in Gesamtmenge von 2 bis 50 Gew.-%, vorzugsweise 5 bis 45 Gew.-%, besonders bevorzugt 10 bis 40 Gew%, mit der Maßgabe, dass das enthaltene Zink(II)-Carboxylat in stöchiometrischer Form eingesetzt wird, also mit Zn(II) und Carboxylat im molaren Verhältnis von 1 zu 2,
ii) optional mindestens ein Trägermedium, in Gesamtmenge von 0 bis 95 Gew.-%, vorzugsweise 10 bis 90 Gew.-%, besonders bevorzugt 20 bis 70 Gew%,
iii) mindestens eine Stickstoff-haltige Verbindung V, welche mindestens zwei N-Atome aufweist und bei der es sich um ein modifiziertes Phenol handelt, vorzugsweise wie in einem der Ansprüche 3 bis 5 definiert, in Gesamtmenge von 1 bis 90 Gew.-%, vorzugsweise 2 bis 60 Gew.-%, besonders bevorzugt 5 bis 50 % Gew-%,
Gew.-% jeweils bezogen auf die gesamte Zubereitung, wobei die Komponenten (i) bis (iii) zusammen vorzugsweise mindestens 51 Gew.-% der gesamten Zubereitung ausmachen müssen,
sowie vorzugsweise zusätzlich umfassend
iv) mindestens ein tertiäres Amin, wie in Anspruch 1 oder 7 definiert, in Mengen von 1 bis 30 Gew-%, bevorzugt 2 bis 25 Gew-%, besonders bevorzugt 5 bis 20 Gew-%,
v) optional mindestens einen zusätzlichen Trimerisierungskatalysator, vorzugsweise wie in Anspruch 8 definiert, in Mengen von 1 bis 90 Gew.-%, vorzugsweise 2 bis 60 Gew.- %, besonders bevorzugt 5 bis 50 % Gew-%, Gew.-% wiederum jeweils bezogen auf die gesamte Zubereitung,
wobei die Komponenten i) bis v) zusammen vorzugsweise mindestens 52 Gew.-% der gesamten Zubereitung ausmachen müssen.

12. Verfahren zur Herstellung von Polyurethan- oder Polyisocyanurat-Schaumstoff, durch Umsetzung einer Polyolkomponente mit einer Polyisocyanatkomponente, **dadurch gekennzeichnet, dass** es unter Einsatz einer Zusammensetzung nach einem der Ansprüche 1 bis 10, oder unter Einsatz einer Zink(II)-Carboxylat-haltigen Zubereitung nach Anspruch 11 erfolgt, wobei es bei Einsatz einer Zink(II)-Carboxylat-haltigen Zubereitung nach Anspruch 11 bevorzugt ist, dass der gesamte Massenanteil an Zink(II)-Carboxylat-haltige Zubereitung am fertigen Polyurethanschaum 0,05 bis 10 Gew.-%, bevorzugt von 0,2 bis 5 Gew.-% beträgt.

13. Verwendung einer Zusammensetzung nach einem der Ansprüche 1 bis 10 oder einer Zubereitung gemäß Anspruch 11 bei der Herstellung von Polyurethan- oder Polyisocyanurat-Schaumstoffen, vorzugsweise Polyurethan- oder Polyisocyanurat-Hartschaumstoffen, bevorzugt zur Verbesserung der Gebrauchseigenschaften des Polyurethan- oder Polyisocyanurat-Schaumstoffs, insbesondere zur Erhöhung der Stauchhärte des Polyurethan- oder Polyisocyanurat-Schaumstoffs zu einem frühen Zeitpunkt, im Vergleich zu Polyurethan- oder Polyisocyanurat-Schaumstoffen, die ohne Zink(II)-Carboxylate hergestellt wurden, Stauchhärte bestimmbar nach DIN EN ISO 844:2014-11.

14. Polyurethan- oder Polyisocyanurat-Schaumstoff, erhältlich durch das Verfahren gemäß Anspruch 13.

15. Verwendung von Polyurethan- oder Polyisocyanurat-Schaumstoffen gemäß Anspruch 14 für Zwecke der thermischen Isolation, vorzugsweise als Dämmplatten und Isolationsmittel sowie für Kühlapparaturen.
